# EUROPEAN PATENT SPECIFICATION

(11) **EP 1 516 186 B1**
(45) Date of publication and mention of the grant of the patent: **17.02.2010**
(21) Application number: 03762073.9
(22) Date of filing: 26.06.2003
(51) Int. Cl.: G01N 33/50, G01N 33/574, G01N 33/68

(54) **A METHOD FOR THE DETECTION OF APOPTOSIS**
VERFAHREN ZUM APOPTOSENACHWEIS
PROCEDE DE DETECTION DE L'APOPTOSE

(30) Priority: 26.06.2002 US 392143 P
(43) Date of publication of application: 23.03.2005
(73) Proprietor: UNIVERSITY OF LOUISVILLE RESEARCH FOUNDATION, INC., Louisville, KY 40202 (US)
(72) Inventor: BATES, Paula, J., Louisville, KY 40207 (US); MI, Yingchang, Tianjin 300221 (CN)
(74) Representative: MacLean, Martin Robert
(86) International application number: PCT/US2003/020167
(87) International publication number: WO 2004/003554

(56) References cited:
- WO-A-00/63250
- WO-A-01/35093
- WO-A-99/06588
- US-A- 6 048 703
- MARTELLI A M ET AL: "Behavior of nucleolar proteins during the course of apoptosis in camptothecin-treated HL60 cells." JOURNAL OF CELLULAR BIOCHEMISTRY. MAY 2000, vol. 78, no. 2, May 2000 (2000-05), pages 264-277, XP002401430 ISSN: 0730-2312
- LICHTENSTEIN A V ET AL: "Circulating nucleic acids and apoptosis." ANNALS OF THE NEW YORK ACADEMY OF SCIENCES. SEP 2001, vol. 945, September 2001 (2001-09), pages 239-249, XP002401431 ISSN: 0077-8923
- ROSEN A ET AL: "Autoantigens as substrates for apoptotic proteases: implications for the pathogenesis of systemic autoimmune disease." CELL DEATH AND DIFFERENTIATION. JAN 1999, vol. 6, no. 1, January 1999 (1999-01), pages 6-12, XP002401432 ISSN: 1350-9047
- HOLDENRIEDER STEFAN ET AL: "Nucleosomes in serum of patients with benign and malignant diseases" INTERNATIONAL JOURNAL OF CANCER, vol. 95, no. 2, 20 March 2001 (2001-03-20), pages 114-120, XP002401433 ISSN: 0020-7136
- MI YINGCHANG ET AL: "Apoptosis in leukemia cells is accompanied by alterations in the levels and localization of nucleolin." THE JOURNAL OF BIOLOGICAL CHEMISTRY. 7 MAR 2003, vol. 278, no. 10, 7 March 2003 (2003-03-07), pages 8572-8579, XP002401434 ISSN: 0021-9258
- ROSENTHAL D.S.: 'Detection of DNA breaks in apoptotic cells utilizing the DNA binding domain of poly(ADP-ribose) polymerase with fluorescence microscopy' NUCLEIC ACIDS RESEARCH vol. 25, no. 7, 1997, pages 1437 - 1441, XP002973516
- MARTELLI A.M.: 'Biochemical and morphological characterization of the nuclear matrix from apoptotic HL-60 cells' J. CELL BIOCHEM. vol. 72, no. 1, January 1999, pages 35 - 46, XP002973517

## Description

### BACKGROUND

Human health revolves on the axis of cell life and cell death. Disruption of the delicate balance between these two extremes often manifests in disease and other conditions. Two processes keep this balance from teetering out of control: cell proliferation and cell death. Whereas necrosis is typically thought of as "accidental" death following injury, apoptosis (programmed cell death) is tightly regulated and a natural part of tissue homeostasis and development. However, un- or mis-regulated apoptosis, like unregulated cell growth (tumors and cancers), is a feature of many diseases and conditions. For example, increased apoptosis is characteristic of Acquired Immunodeficiency Syndrome (AIDS); neurodegenerative diseases such as Alzheimer's, Parkinson's, and amyotrophic lateral sclerosis; ischemic injury after myocardial infarction, stroke, and reperfusion; acute inflammatory conditions and sepsis; and in autoimmune diseases such as hepatitis and transplant immunorejection. At the other extreme, decreased apoptosis is an attribute of many malignancies, autoimmune disorders, and some viral infections. Interestingly, insufficiencies in the apoptotic program, possibly by failure to eliminate autoreactive T-cells or inefficient clearance of apoptotic material ironically leads to the *development* of autoimmune diseases, such as Hashimoto's thyroiditis, ulcerative colitis, type I diabetes mellitus and systemic lupus erythematosus.

A hallmark of cancer cells is not only uncontrolled proliferation, but also a decreased rate of apoptosis. This attribute can confound treatments that induce apoptotic pathways to kill cancer cells. For example, a common cause of leukemia treatment failure is the development of chemotherapy-resistant disease; this drug-resistant phenotype often correlates with molecular defects in the apoptotic cellular pathways. Elucidation of the mechanisms controlling apoptosis induction and subsequent cellular disintegration would result in improved methods for the diagnosis of chemotherapy-resistant cancers.

When a cell undergoes apoptosis, the structure of the cell breaks down. The breakdown components are packaged into apoptotic bodies, membrane bound "sacs" that contain nucleic acids, proteins and lipids. Usually, macrophages or neighboring cells engulf these bodies, clearing them from the system. However, when the ability of neighboring cells and/or macrophages are overwhelmed by high numbers of bodies ("excessive" apoptosis) or defects in clearing the bodies, apoptotic bodies are released into circulation and can be detected in blood plasma or serum (Holdenrieder *et al*., 2001a; Holdenrieder *et al*., 2001b; Holdenrieder *et al*., 2001c; Lichtenstein *et al*., 2001).

Above-average levels of apoptotic bodies in the bloodstream have been correlated with the presence tumors and cancers. While this statement appears to contradict the general observation that apoptotic levels are decreased in tumor and cancer cells, the statement is not absolute. Resistance to apoptosis is usually a late event in malignant progression-that is, resistance to apoptosis increases as the cancer grows and becomes metastatic. Therefore, early stage tumors can be characterized by slow overall growth, reflecting a high proliferation rate balanced by a high level of apoptosis. Even in late stage tumors with relatively low rates of apoptosis, the absolute number of apoptotic bodies can be high due to the large tumor mass.

### Nucleolin

Nucleolin (Bandman *et al*., 1999) is an abundant, non-ribosomal protein of the nucleolus, the site of ribosomal gene transcription and packaging of pre-ribosomal RNA. This 707 amino acid phosphoprotein has a multi-domain structure consisting of a histone-like N-terminus, a central domain containing four RNA recognition motifs and a glycine/arginine-rich C-terminus and has an apparent molecular weight of 110 kD. Its multiple domain structure reflects the remarkably diverse functions of this multifaceted protein (Ginisty *et al*., 1999; Srivastava and Pollard, 1999; Tuteja and Tuteja, 1998). Nucleolin has been implicated in many fundamental aspects of cell survival and proliferation. Most understood is the role of nucleolin in ribosome biogenesis. Other functions may include nucleocytoplasmic transport, cytokinesis, nucleogenesis and apoptosis.

Nucleolin synthesis has been correlated with increased rates of cell division (cell proliferation); nucleolin levels are therefore higher in tumor and cancer cells compared to most normal cells (Tuteja and Tuteja, 1998). Nucleolin is one of the nuclear organizer region (NOR) proteins whose levels, as measured by silver staining ofbiopsied samples, are assessed by pathologists as a marker of cell proliferation and an indicator of malignancy (Derenzini, 2000).

Also present in the cell plasma membrane in a limited number of cell types, such as lymphocytes and inner medullary collecting duct cells, nucleolin has been hypothesized to function as a receptor (*e.g*., (Callebaut *et al*., 1998; Sorokina and Kleinman, 1999)). The expression of plasma membrane nucleolin is most often seen in neopolastic cells (such as malignant or pre-malignant). In addition, a correlation between nucleolin plasma membrane expression and the aggressiveness of neoplastic disease has been identified.

### Detecting apoptosis

Apoptosis has been detected by a variety of accepted methods (Siman *et al*., 2000), using morphology, DNA fragmentation, enzymatic activity, and polypeptide degradation. In some morphological assays, methods usually exploit nuclear chromatin condensation and the fragmentation of nuclear structures into apoptotic bodies. These changes can be observed using conventional stains and dyes that selectively accumulate in nuclei; or they can be observed morphologically at the ultrastructural level. Some enzymatic-activity based methods use those enzymes specific to apoptosis, such as caspase 9 and caspase-3 (Martin and Green, 1995; Thornberry and Lazebnik, 1998; Zou *et al.,* 2001).

Nucleic acid-based methods use DNA fragmentation that is characteristic of apoptosis. When resolved using electrophoresis on agarose gels, apoptotic DNA initially has a characteristic "ladder" pattern, as opposed to a smear of nucleic acids that is observed, for example, in necrosis or other non-specific DNA degradation. A common histochemical technique to detect DNA fragmentation uses end-labeled DNA. Kits for such are commercially available, such as the APOLERT DNA fragmentation kit (Clontech Laboratories, Inc.; Palo Alto, CA). This assay is based on terminal deoxynuclotidyltransferase (Tdt)-mediated dUTP nick-end labeling (TUNEL), where Tdt catalyzes the incorporation of fluorescein-dUTP at the free 3'-hydroxyl ends of fragmented DNA in cells undergoing apoptosis.

Proteolysis of specific cellular proteins associated with apoptosis can also be used. For example, poly(ADP-ribose) polymerase (PARP-1) is specifically cleaved during apoptosis. PARP-1 is a DNA-binding protein that catalyzes the addition of poly(ADP-ribose) chains to some nuclear proteins and is thought to play a critical role in DNA damage repair. PARP-1 is rapidly activated during cellular stresses, such as heat shock, ionizing radiation, exposure to carcinogens, and treatment with chemotherapy agents (Scovassi and Poirier, 1999; Wyllie *et al*., 1980). During apoptosis caspase-3 cleaves PARP-1; in fact, the resolution of the 89 kD and 24 kD proteolytic fragments is accepted as a hallmarks of apoptosis (Scovassi and Poirier, 1999; Wyllie *et al*., 1980).

### Apoptotic bodies in disease and neoplastic cells (cancer and tumor cells)

Cancer, inflammatory diseases and autoimmune disease are associated with defects in apoptosis. For example, apoptotic bodies are observed in various forms of cancer, such as endocervical adenocarcinomas, prostatic carcinomas, breast cancers, leukemias and non-small cell lung carcinomas. In addition, the mean number of apoptotic bodies present has been correlated to the progression of cancer (Biscotti and Hart, 1998; Choi *et al*., 1999; Sohn *et al*., 2000; Tormanen *et al*., 1995).

Chemotherapy and radiotherapy treatment often induce high levels of apoptosis. However, neoplastic cells may be resistant to treatment. For example, in the case of leukemia, particularly acute leukemias, failure of malignant cells to undergo cell death in response to chemotherapy, is a major cause of treatment failure (Schimmer *et al*., 2001). In many cases, chemoresistance is associated with aberrant expression of the proteins involved in the activation and regulation of apoptosis. Consequently, levels of apoptosis-associated proteins are important prognosticators in the clinical management of acute leukemia's, and several therapeutic strategies based on modulating apoptotic pathways are currently in development (Pinton *et al*., 2001; Schimmer *et al*., 2001; Sutton *et al*., 2000). The success of cancer treatments depend in part on its early detection. As such, methods that are capable of indicating neoplasms at the earliest stages are needed. During cancer therapy, especially in the case of chemotherapy-resistant disease, a means to detect chemotherapy resistant cells as well as a means to evaluate treatment effectiveness would be invaluable allies in the war on cancer.

Martelli A.M. et al., J. Cellular Biochemistry, May 2000, Vol.78, No. 2, pages 264-277, describes the behaviour of nucleolar proteins during the course of apoptosis in camptothecin-treated cells.

Lichtenstein A.V. et al., Ann. New York Acad. Sci., Sept 2001, Vol. 945, page 239-249, describes circulating nucleic aids and apoptosis.

US 6,048,703 describes methods for detecting cell apoptosis.

Rosen A. et al., Cell Death and Differentiation, Jan. 1999, Vol. 6, No. 1, pages 6-12, describes auto-antigens as substrates for apoptotic proteases: implications for the pathogenesis of systemic autoimmune disease.

WO 00/63250 describes antibodies that recognize APP cleaved by caspases and methods of use thereof.

WO 01/35093 describes a method of detecting cell death and a detection reagent.

WO 99/06588 describes a cell-free system of mitochondria-dependent apoptosis and methods of use thereof.

Holdenrieder S. et al., Int. J. Cancer, Vol. 95, No. 2, 20 March 2001, pages 114-120, describes nucleosomes in the serum of patients with benign and malignant diseases.

Mi Y. et al., J. Biol. Chem., 7 March 2003, Vol. 278, No. 10, pages 8572-8579, describes that apoptosis in leukaemia cells is accompanied by alterations in the levels and localization of nucleolin.

The present invention provides a method of detecting apoptosis, comprising: detecting at least one of nucleolin and poly(ADP-ribose) polymerase in or from apoptotic bodies in a sample, wherein said sample is a sample from which cells have been removed.

Examples of samples that may be rendered cell free include, but are not limited to blood, tissue, tissue culture media and sputum. In some cases, detection is facilitated by disrupting the membranes of apoptotic bodies in the sample. Antibodies and oligonucleotides that bind nucleolin or PARP-1 may be used for detection.

The method of detecting apoptosis may be for detecting excessive apoptosis in a subject.

The subject may be suffering from Acquired Immunodeficiency Syndrome, a neurodegenerative disease, an ischemic injury, an autoimmune disease, a tumour, a cancer, a viral infection, acute inflammatory conditions and sepsis. The cancers from which a subject may suffer include, but are not limited to, endocervical adenocarcinoma, prostatic carcinoma, breast cancer, leukaemia and non-small lung carcinoma.

In another aspect, the invention provides a method of determining if a compound induces apoptosis, comprising: contacting a cell with the compound; and detecting apoptosis by the method described above.
Figure 1 shows immunofluorescence staining of nucleolin in U937 cells.
Figure 2 shows nucleolin found in the medium of untreated and apoptotic U937 cells *in vitro.*

Nucleolin has been discovered to be an unexpectedly convenient and reliable marker for the detection of apoptotic bodies, especially those shed into circulation. Detecting nucleolin in the circulation, such as in isolated plasma or serum, correlates with levels of apoptosis that overwhelm the usual apoptotic body-clearing cells, such as macrophages and/or neighboring cells to the site of apoptosis. The presence of cancers and tumors, as well as other conditions such as autoimmune diseases, has been correlated with high numbers of apoptotic bodies in the circulation. The detection of apoptotic bodies therefore may facilitate the early detection of diseases characterized by apoptotic cell death, especially malignant diseases; as well as a method to monitor disease progression and therapeutic intervention effectiveness.

Nucleolin decreases in the nucleus and mis-localizes to the plasma membrane in neoplastic cells, enabling for the detection of apoptotic bodies shed into circulation. Sinc nucleolin is found in every nucleated cell, a convenient method for the detection of apoptotic bodies is the use of nucleolin as a marker, providing a useful method for the early detection of diseases characterized by apoptotic cell death. In addition, disease progression and evaluation of therapeutic response maybe assessed using nucleolin to detect apoptosis. Such techniques are also useful in the screening for potential therapeutic agents that may induce or prevent apoptosis.

The advantages of detecting nucleolin in apoptotic bodies include:
1. Facilitated detection of cancers and tumors. Serum-based cancer markers are currently only available for certain cancers (e.g. prostate cancer (Prostate Specific Antigen (PSA)) and ovarian cancer (Ca-125)). Since cancers and tumors can undergo apoptosis at rates that overwhelm the endogenous clearing mechanisms, allowing for the introduction of apoptotic bodies into the circulation, the detection of nucleolin to identify apoptotic bodies provides for a test allowing for the detection of cancers and tumors. Such a test method allows for the detection of a wide range of cancers and tumors, acting as a universal detection marker.
2. Easier, more convenient testing. Current approaches for detecting apoptotic bodies detect circulating RNA or DNA. To ensure detection, these sequences need to be often amplified *in vitro.* Such amplification procedures are highly sensitive to sample contamination. When detecting nucleolin, the sample is processed using protocols that are less sensitive to contaminants.
3. Greater sensitivity. Experimental approaches involving the detection of circulating cancer cells tend not to be sensitive due to the relatively small number of such cells in the circulation as compared to the relatively high number of apoptotic bodies under the same conditions.

### Definitions

"Apoptosis" refers to cell death by an intracellular controlled process characterized by a condensation and subsequent fragmentation of the cell nucleus during which the plasma membrane remains intact.

An "apoptotic body" contains nucleic acids, proteins, lipids, but no nucleus, although it may contain fragmented nuclei. In general, apoptotic bodies are ≤ 10 µm, preferably between 0.2 µm ≤ 8 µm, and more preferably, 0.2 µm ≤ 0.45 µm.

A "neoplasm" is an abnormal tissue growth resulting from neoplastic cells, cells that proliferate more rapidly and uncontrollably than normal cells. Usually partially or completely structurally disorganized, neoplasms lack functional coordination with the corresponding normal tissue. Neoplasms usually form a distinct tissue mass that may be either benign (tumor) or malignant (cancer).

"Cancer cells" invade surrounding tissues, may metastasize to distant sites, and are likely to recur after attempted removal, causing death of a subject if not adequately treated. In addition to structural disorganization, cancer cells usually regress to more primitive or undifferentiated states (anaplasia), although morphologically and biochemically, they may still exhibit many functions of the corresponding wild-type cells. Carcinomas are cancers derived from epithelia; sarcomas are derived from connective tissues.

Cancers may be more aggressive or less aggressive. The aggressive phenotype of a cancer cell refers to the proliferation rate and the ability to form tumors and metastasize in nude mice. Aggressive cancers proliferate more quickly, more easily form tumors and metastasize than less-aggressive tumors.

"Neoplastic state" refers to three conditions: normal, pre-malignant and malignant. "Normal" refers to a growth or cell that is clinically normal (healthy). "Pre-malignant" refers to a growth or cell that is on the pathway to malignancy, but at the time of examination, would not be classified as malignant by conventional methods. "Malignant" refers to a cell or growth that has at least one of the following properties: locally invasive, destructive growth and metastasis.

"Removing cells" from a sample means to remove cells in such a way as to prevent access to nucleolin in the cells. For example, most detergent extractions would destroy cellular integrity, but nucleolin would also be freed from the nucleus. Physical separations, such as centrifugation, affinity purifications, *etc*., are good techniques for removing cells from a sample.

### GROs and other polypeptide-binding oligonucleotides

Oligonucleotides are available that specifically bind to polypeptides, such as nucleolins. Examples of such are GROs, which are guanosine-rich oligonucleotides. Characteristics of GROs include:
(1) having at least 1 GGT motif
(2) preferably having 4-100 nucleotides, although GROs having many more nucleotides are possible
(3) having chemical modifications to improve stability.

Especially useful GROs form G-quartet structures, as indicated by a reversible thermal denaturation/renaturation profile at 295 nm (Bates *et al*., 1999). Preferred GROs also compete with a telomere oligonucleotide for binding to a target cellular protein in an electrophoretic mobility shift assay (Bates *et al*., 1999). GROs, like other polynucleotides, can be derivatized to carry a detectable label.

Other oligonucleotides may have high binding specificity for nucleolin.

### anti-nucleolin agent

An "anti-nucleolin agent" binds to nucleolin. Examples include anti-nucleolin antibodies and certain oligonucleotides.

### Embodiments

The following embodiments are given as non-limiting examples of various ways to practice the invention.

In all embodiments, the underlying principle is to detect the presence of nucleolin in or from apoptotic bodies. Detection techniques wherein nucleolin-detecting reagents have access to interior portions of the apoptotic body are useful, as are techniques wherein nucleolin is extracted from the apoptotic body before detection.

In an embodiment, nucleolin is detected within an apoptotic body. An apoptotic body isolated from a subject is treated with an agent to allow a nucleolin-binding reagent access to nucleolin in the body. The nucleolin in or from the apoptotic body is then contacted with the nucleolin-binding reagent.

An isolated apoptotic body, or a sample containing apoptotic bodies, may comprise a larger tissue sample. Alternatively, a blood, sputum or other physiological fluid is from a subject. Detection procedures may use anti-nucleolin antibodies; these antibodies may be directly labeled or when bound, detected indirectly. Other useful nucleolin detection agents include GROs that specifically bind nucleolin. Procedures, such as fluorescence-activated cell sorting (FACS; adapted for apoptotic bodies as necessary) or immunofluorescence, employ fluorescent labels, while other cytological techniques, such as histochemical, immunohistochemical and other microscopic (electron microscopy (EM), immuno-BM) techniques use various other labels, including colorimetric and radioactive labels. The various reagents may be assembled into kits.

In another embodiment, isolated apoptotic bodies may be disrupted to release nucleolin, and the nucleolin detected using an agent that binds nucleolin. Such a technique is particularly useful for detecting nucleolin in a blood, sputum or other fluid sample from a subject. Techniques to detect nucleolin include those wherein the extracted nucleolin is placed on a substrate, and the substrate is then probed with a nucleolin-detecting reagent. Examples of such techniques include polypeptide dot blots and immuno- (Western blots), biochips, protein arrays, *etc.*. Other detection formats include enzyme-linked immunosorbent assays (ELISAs) and related techniques (Ausubel, 1987). The various reagents may be assembled into kits.

A sample containing apoptotic bodies may be from a subject and the nucleolin contained in this sample may be detected. The following, not meant to limit the invention, is presented to aid the practitioner in carrying out the invention, although other methods, techniques, reagents and approaches can be used to achieve the invention.

### Sample preparation

Cells or tissue samples are from a subject. The subject is a vertebrate, more preferably a mammal, such as a monkey, dog, cat, rabbit, cow, pig, goat, sheep, horse, rat, mouse, guinea pig, *etc*.; and most preferably a human. Techniques to collect a desired sample include biopsy, surgery, scraping (inner cheek, skin, *etc*.) and blood withdrawal.

Under conditions of excessive apoptosis, that is, programmed cell death that overwhelms the usual apoptotic body clearing mechanisms, apoptotic bodies are released into the circulation. For those methods that involve the detection of apoptotic bodies in blood, blood cells (especially leukocytes) may be removed. Antibody-based methods or other techniques may then be used to detect nucleolin from/in these bodies by measuring nucleolin in serum (wherein coagulation has occurred and the coagulated material removed) or plasma (the fluid part of blood without any special treatment. Both serum and plasma are substantially cell-free. Either fresh blood plasma or serum, or archived serum or plasma, such as by freezing or lyophilization, may be used. Blood can be drawn by standard methods of venepuncture and collected into a collection tube, preferably siliconized glass. Blood collection in the absence of anticoagulant reagents allow for the preparation of serum; anticoagulants, such as Ethylenediaminetetraacetic (EDTA), citrate (*e.g*., sodium citrate), or heparin are used to prepare plasma. Serum or plasma are isolated from whole blood via a variety of techniques. These include centrifugation, using preferably gentle centrifugation at 300-800g for five to ten minutes. As an alternative to centrifugation, filtration-based separation techniques may be used to separate serum or plasma. A filter that may be used to separate a sample into a cell-containing fraction and an apoptotic body-containing sample may comprise two membranes; wherein one membrane removes undesired materials (such as cells), while the second membrane traps desired materials, such as apoptotic bodies, thus allowing for the simultaneous fractionation and concentration of the desired materials.

For those methods that analyze certain conditions, such as lung carcinomas, sputum collection is a convenient and easily obtained sample collection technique. "Sputum" refers to expectorated matter comprising saliva and discharges from the respiratory airways. Sputum is a highly complex material that has a pronounced gel-like structure. For collection of sputum, Byrne *et al*. (Byrne, 1986) suggest that the patient collect material, raised by several deep coughs, in a container with a lid. Alternatively, sputum can be collected by using a bronchoscope (Kim *et al*., 1982). Specific devices or agents may be used to facilitate sputum collection (Babkes *et al*., 2001; King and Speert, 2002; Rubin and Newhouse, 1999). Sputum samples, like any other physiological sample, can be rendered cell-free, using, for example, physical separations (such as centrifugation, with or without gradients, or filtration).

### Detecting nucleolin and PARP-1: antibody-based methods

Apoptotic bodies containing nucleolin and PARP-1 can be detected in samples including cells, tissue sections, cell cultures, and blood. Immunochemical methods to detect protein expression, such as nucleolin or PARP-1 proteins, are well known and include Western blotting, immunoaffinity purification, immunoprecipitation, enzyme-linked immunosorbent assay (ELISA), dot or slot blotting, radioimmunoassay (RIA), fluorescent immunoassay, chemiluminescent immunoassay (CMIA), immunohistochemical detection, immunocytochemical staining, and flow cytometry. Common procedures and instructions using antibodies have been well addressed (*e*.*g*., (Harlow and Lane, 1988; Harlow and Lane, 1999). Selected antibodies that are useful for detecting nucleolin are shown in Table 1A; those for detecting PARP-1 are shown in Table 1B.

**Table 1A Anti-nucleolin antibodies**

| **Antibody** | **Source** | **Antigen source** | **Notes** |
|---|---|---|---|
| p7-lA4 mouse monoclonal antibody (mAb) | Developmental Studies Hybridoma Bank (University of Iowa; Ames, IA) | *Xenopus laevis* oocytes | IgG₁ |
| sc-8031 mouse mAb | Santa Cruz Biotech (Santa Cruz, CA) | human | IgG₁ |
| sc-9893 goat polyclonal Ab (pAb) | Santa Cruz Biotech | human | IgG |
| sc-9892 goat pAb | Santa Cruz Biotech | human | IgG |
| clone 4E2 mouse mAb | MBL International (Watertown, MA) | human | IgG₁ |
| clone 3G4B2 mouse mAb | Upstate Biotechnology (Lake Placid, NY) | dog (MDCK cells) | IgG₁ₖ |

**Table 1B Anti-PARP-1 antibodies**

| **Antibody** | **Sources** | **Antigen source** | **Notes** |
|---|---|---|---|
| sc-1562 goat pAb | Santa Cruz Biotech | Mouse amino terminus | Reacts with both cleaved products; IgG |
| sc-8007 mouse mAb | Santa Cruz Biotech | Human, 764-2024 carboxy residues | IgG₂ₐ |
| sc-1561 goat pAb | Santa Cruz Biotech | Human (?), amino terminus | Reacts with both cleaved products; IgG. |
| sc-1561-Y chicken pAb | Santa Cruz Biotech | Human (?), amino terminus | React with both cleaved products; IgY. Same as sc1561, except chicken is host animal |
| sc-7150 rabbit pAb | Santa Cruz Biotech | Human, 764-2024 carboxy residues | IgG; reacts with both cleaved products. |

If additional anti-nucleolin or PARP-1 antibodies are desired, they can be produced using well-known methods (Harlow and Lane, 1988; Harlow and Lane, 1999). For example, polyclonal antibodies can be raised in a mammalian host by one or more injections of an immunogen, such as an extracellular domain of surface-expressed nucleolin, and if desired, an adjuvant. Typically, the immunogen (and adjuvant) is injected in a mammal by a subcutaneous or intraperitoneal injection. The immunogen may include components such as polypeptides (isolated, non-isolated, or recombinantly produced), cells or cell fractions. Examples of adjuvants include Freund's complete, Freund's incomplete, and monophosphoryl Lipid A synthetic-trehalose dicorynomycolate (MPL-TDM). To improve the immune response, an immunogen may be conjugated to a polypeptide that is immunogenic in the host, such as keyhole limpet hemocyanin (KLH), serum albumin, bovine thyroglobulin or soybean trypsin inhibitor. Alternatively, polyclonal antibodies may be made in chickens, producing IgY molecules (Schade *et. al*., 1996).

Monoclonal antibodies may also be made by immunizing a host or lymphocytes from a host, harvesting the monoclonal antibody-secreting (or potentially secreting) lymphocytes, fusing those lymphocytes to immortalized cells (*e.g*., myeloma cells), and selecting those cells that secrete the desired monoclonal antibody (Goding, 1996). If desired, the monoclonal antibodies may be purified from the culture medium or ascites fluid by conventional procedures such as protein A-sepharose, hydroxylapatite chromatography, gel electrophoresis, dialysis, ammonium sulfate precipitation or affinity chromatography (Harlow and Lane, 1988; Harlow and Lane, 1999). The antibodies may be whole antibodies and fragments or derivatives thereof.

An approach using antibodies to detect the presence of an antigen usually include one or more of the following steps:
(1) attaching the entity being tested for an antigen, such as nucleolin or PARP-1, to an appropriate substrate;
(2) preparing the entity being tested for the antigen by washing with buffer or water;
(3) blocking non-specific antibody binding sites;
(4) applying the antibody (*e.g*., nucleolin or PARP-1 antibody); and
(5) detecting bound antibody, either via a detectable labeled-secondary antibody that recognizes the primary antibody or a detectable label that has been directly attached to, or associated with, the bound (anti-nucleolin or PARP-1) antibody.

Substrates may be washed with any solution that does not interfere with epitope structure. Common buffers include saline and biological buffers, such as bicine, tricine, and Tris.

Non-specific binding sites are blocked by applying a protein solution, such as bovine serum albumin (BSA; denatured or native), milk proteins, or in the cases wherein the detecting reagent is a secondary antibody, normal serum or immunoglobulins from a non-immunized host animal whose species is the same origin as the detecting antibody. For example, a procedure using a secondary antibody made in goats would employ normal goat serum (NGS).

The substrate is then reacted with the antibody of interest. The antibody may be applied in any form, such as F_{ab} fragments and derivatives thereof, purified antibody (by affinity, precipitation, *etc*.), supernatant from hybridoma cultures, ascites, serum or recombinant antibodies expressed by recombinant cells. The antibody may be diluted in buffer or media, often with a protein carrier such as the solution used to block non-specific binding sites; the useful antibody concentration is usually determined empirically. In general, polyclonal sera, purified antibodies and ascites may be diluted 1:50 to 1:200,000, more often, 1:200 to 1:500. Hybridoma supernatants may be diluted 1:0 to 1:10, or may be concentrated by dialysis or ammonium sulfate precipitation (or any other method that retains the antibodies of interest but at least partially removes the liquid component and preferably other small molecules, such as salts) and diluted if necessary. Incubation with antibodies may be carried out for as little as 20 minutes at 37° C, 1 to 6 hours at room temperature (approximately 22° C), or 8 hours or more at 4°C.

To detect an antibody-antigen complex, a label may be used. The label may be coupled to the binding antibody or to a second antibody that recognizes the first antibody and is incubated with the sample after the primary antibody incubation and thorough washing. Suitable labels include fluorescent moieties, such as fluorescein isothiocyanate; fluorescein dichlorotriazine and fluorinated analogs of fluorescein; naphthofluorescein carboxylic acid and its succinimidyl ester; carboxyrhodamine 6G; pyridyloxazole derivatives; Cy2, 3 and 5; phycoerythrin; fluorescent species of succinimidyl esters, carboxylic acids, isothiocyanates, sulfonyl chlorides, and dansyl chlorides, including propionic acid succinimidyl esters, and pentanoic acid succinimidyl esters; succinimidyl esters of carboxytetramethylrhodamine; rhodamine Red-X succinimidyl ester; Texas Red sulfonyl chloride; Texas Red-X succinimidyl ester; Texas Red-X sodium tetrafluorophenol ester; Red-X; Texas Red dyes; tetramethylrhodamine; lissamine rhodamine B; tetramethylrhodamine; tetramethylrhodamine isothiocyanate; naphthofluoresceins; coumarin derivatives; pyrenes; pyridyloxazole derivatives; dapoxyl dyes; Cascade Blue and Yellow dyes; benzofuran isothiocyanates; sodium tetrafluorophenols; 4,4-difluoro-4-bora-3a,4a-diaza-*s*-indacene. Suitable labels further include enzymatic moieties, such as alkaline phosphatase or horseradish peroxidase; radioactive moieties, including ³⁵S and ¹³⁵I-labels; avidin (or streptavidin)-biotin-based detection systems (often coupled with enzymatic or gold signal systems); and gold particles. In the case of enzymatic-based detection systems, the enzyme is reacted with an appropriate substrate, such as 3, 3'-diaminobenzidine (DAB) for horseradish peroxidase; preferably, the reaction products are insoluble. Gold-labeled samples, if not prepared for ultrastructural analyses, may be chemically reacted to enhance the gold signal; this approach is especially desirable for light microscopy. The choice of the label depends on the application, the desired resolution and the desired observation methods. For fluorescent labels, the fluorophore is excited with the appropriate wavelength and the sample observed using a microscope, confocal microscope, or FACS machine. In the case of radioactive labeling, the samples are contacted with autoradiography film, and the film developed; alternatively, autoradiography may also be accomplished using ultrastructural approaches. Alternatively, radioactivity may be quantified using a scintillation counter.

### Morphological-coupled approaches:

The presence of nucleolin and/or PARP-1 in apoptotic bodies can be ascertained by immunolocalization. Generally, the apoptotic bodies, or cells or tissue containing such bodies are preserved by fixation, exposed to an antibody that recognizes the antigen of interest, such as nucleolin or PARP-1, and the bound antibody visualized.

Any tissue or even an entire organism is appropriate for fixation. Tissue may be from any organ, plant or animal, and may be harvested after or prior to fixation. Alternatively, a blood sample may be obtained, and serum or plasma prepared. Separation conditions may be chosen to ensure that apoptotic bodies are separated out from any blood cells. Apoptotic bodies may then be visualized using a cytological-based technique.

Fixation, if desired, may be by any known means; the requirements are that the protein to be detected is not rendered unrecognizable by the binding agent, most often an antibody. Appropriate fixatives include paraformaldehyde-lysine-periodate, formalin, paraformaldehyde, methanol, acetic acid-methanol, glutaraldehyde, acetone, Kamovsky's fixative, *etc*. The choice of fixative depends on variables such as the protein of interest, the properties of a particular detecting reagent (such as an antibody), the method of detection (fluorescence, enzymatic) and the method of observation (epifluorescence microscopy, confocal microscopy, light microscopy, electron microscopy, *etc*.). The sample is usually first washed, most often with a biological buffer, prior to fixation. Fixatives are prepared in solution or in biological buffers; many fixatives are prepared immediately prior to applying to the sample. Suitable biological buffers include saline (*e.g*., phosphate buffered saline), N-(carbamoylmethyl)-2-aminoethanesulfonic acid (ACES), N-2-acetamido-2-iminodiacetic acid (ADA), bicine, bis-tris, 3-cyclohexylamino-2-hydroxy-1-propanesulfonic acid (CAPSO), ethanolamines, glycine, N-2-hydroxyethylpiperazine-N'-2-ethanesulfonic acid (HEPES), 2-N-morpholinoethanesulfonic acid (MES), 3-N-morpholinopropanesulfonic acid (MOPS), 3-N-morpholino-2-hyrdoxy-propanesulfonic acid (MOPSO), piperazine-N,N'-bis(2-ethanesulfonic acid) (PIPES), tricine, triethanolamine, *etc*. An appropriate buffer is selected according to the sample being analyzed, appropriate pH, and the requirements of the detection method. A useful buffer is phosphate buffered saline (PBS). After fixation, the sample may be stored in fixative, preferably fresh, or temporarily or indefinitely, at a temperature between about 4° C to about 22° C. In some cases, depending on the characteristics of the sample, the sample may be attached to a substrate, such as to a glass coverslip, microscope slide or plastic. Such substrates may be treated to enhance attachment; such treatments included charging the substrate, coating the substrate with an adhesive material, such as poly-(L or D or combination)-lysine, extracellular matrix molecules or compositions, *etc*.

After fixation from 5 minutes to 1 week, depending on the sample size, sample thickness, and viscosity of the fixative, the sample is washed in buffer. If the sample is thick or sections are desired, the sample may be embedded in a suitable matrix. For cryosectioning, sucrose is infused, and embedded in a matrix, such as OCT Tissue Tek (Andwin Scientific; Canoga Park, CA) or gelatin. Samples may also be embedded in paraffin wax, or resins suitable for electron microscopy, such as epoxy-based (Araldite, Polybed 812, Durcupan ACM, Quetol, Spurr's, or mixtures thereof; Polysciences, Warrington, PA), acrylates (London Resins (LR White, LR gold), Lowicryls, Unicryl; Polysciences), methylacrylates (JB-4, OsteoBed; Polysciences), melamine (Nanoplast; Polysciences) and other media, such as DGD, Immuno-Bed (Polysciences) and then polymerized. Resins that are especially appropriate include hydrophilic resins (such as Lowicryls, London Resins, water-soluble Durcupan, *etc*.) since these are less likely to denature the protein of interest during polymerization and will not repel antibody solutions. When embedded in wax or resin, samples are dehydrated by passing them through a concentration series of ethanol or methanol; in some cases, other solvents may be used, such as polypropylene oxide. Embedding may occur after the sample has been reacted with the detecting agents, or samples may be first embedded, sectioned (via microtome, cyrotome, or ultramicrotome), and then the sections reacted with the detecting reagents. In some cases, the embedding material may be partially or completely removed before detection to facilitate antigen access.

In some instances, the nucleolin or PARP-1 epitope(s) to which the antibody binds may be rendered unavailable because of fixation. Antigen retrieval methods can be used to make the antigen available for antibody binding. Many recourses are available (reviewed in, for example, (Holdenrieder *et al*., 2001b; McNicol and Richmond, 1998; Robinson and Vandre, 2001)). Common methods include using heat supplied from autoclaves, microwaves, hot water or buffers, pressure cookers, or other sources of heat. Often the sources of heat are used in sequence; the samples must often be in solution (*e.g*., microwave treatments). Detergent treatment may also unmask antigens, such as sodium dodecyl sulfate (SDS, 0.25% to 1%) or other denaturing detergents. Chemical methods include strong alkalis (such as NaOH), prolonged immersion in water, urea, formic acid and refixation in zinc sulfate-formalin. In other instances, proteolytic enzyme treatment will modify the antigen such that it is available to the antibody. Any number of proteases may be used, such as trypsin. These methods may be combined to achieve optimal results. The choice of the antigen retrieval method will depend on the sample, its embedment (if any), and the anti-nucleolin or PARP-1 antibody.

Especially in the cases of immunofluorescent or enzymatic product-based detection, background signal due to residual fixative, protein cross-linking, protein precipitation or endogenous enzymes may be quenched, using, *e.g*., ammonium chloride or sodium borohydride or a substance to deactivate or deplete confounding endogenous enzymes, such as hydrogen peroxide which acts on peroxidases. To detect intracellular proteins in samples that are not to be sectioned, samples may be permeabilized. Permeabilizing agents include detergents, such as t-octylphenoxypolyethoxyethanols, polyoxyethylenesorbitans, and other agents, such as lysins, proteases, *etc*.

Non-specific binding sites are blocked by applying a protein solution, such as bovine serum albumin (BSA; denatured or native), milk proteins, or preferably in the cases wherein the detecting reagent is an antibody, normal serum or IgG from a non-immunized host animal whose species is the same is the same origin of the detecting antibody.

### Flow cytometry/Fluorescence-Activated Cell Sorting (FACS)

Methods of performing flow cytometry are well known (Orfao and Ruiz-Arguelles, 1996). After harvesting, preparations containing apoptotic bodies are prepared as a single-body suspension; the apoptotic bodies are then incubated with an anti-nucleolin or PARP-1 antibody usually after blocking non-specific binding sites. Preferably, the anti-nucleolin or PARP-1 antibody is labeled with a fluorescent marker. If the antibody is not labeled with a fluorescent marker, a second antibody that is immunoreactive with the first antibody and contains a fluorescent marker can be used. After sufficient washing to ensure that excess or unbound antibodies are removed, the preparation is ready for flow cytometry.

### Biochemical assay-based approaches:

Apoptotic bodies may be released into the circulation and detected in the blood. Immunochemical or other techniques may be used to detect these bodies by measuring nucleolin and/or PARP-1 in serum or plasma obtained from a subject. In these approaches, it may be desirable to release nucleolin and/or PARP-1 by disrupting the apoptotic bodies before detection of nucleolin or PARP-1. This may be achieved in any number of ways, such as simple cell extraction, differential extraction or mechanical disruption. Extracting reagents are well known. For example, solvents such as methanol may be occasionally useful. More likely, detergents, such as t-octylphenoxypolyethoxyethanol (also known as polyethylene glycol tert-octylphenyl ether) are particularly useful for simple extractions. Also useful are glucopyranosides, maltopyranosides, maltosides, polyoxyethylene esters, other polyoxyethylene ethers, salts of alginic, caprylic, cholic 1-decanesulfonic, deoxycholic, dioctyl sulfosuccinate, 1-dodecanesulfonic, glyocholic, glycodeoxycholic, 1-heptanesulfonic, 1-hexanesulfonic, N-lauroylsacrosine, lauryl sulfate (*e.g*., SDS), 1-nonanesulfonic, 1-octanesulfonic, 1-pentanesulfonic, taurocholic and tauodexycholic acids; sodium 7-ethyl-2-methyl-4-undecyl sulfate, and sodium 2-ethylhexyl sulfate. Other useful detergents included(3-{(3-cholamidopropyl)dimethylammonio}-1-propane-sulfonate, (3-{(3-cholamidopropyl)dimethylammonio}-2-hydroxy-1-propane-sulfonate, N-decyl-, N-dodecyl-, N-hexadecyl-, N-octadecyl-, N-tetradecyl-N,N-dimethyl-3-ammonio-1-propanesulfonates and phosphatidylcholine. Less useful, but may be helpful in some cases, are alkyltrimethylammonium bromides, benzalkonium chloride, benzethonium chloride, benzyldimethyldodecylammonium bromide, benzyldimethylhexadecylammonium chloride, cetyldimethylethylammonium bromide, cetylpyridinium, decamethonium bormide, dimethyldioctadecylammonium bromide, methylbenzethonium chloride, methyltiroctylammonium chloride, and N,N',N'-polyoxyehtlylene(10)-N-tallow-1,3-diaminopropane. The different extracting reagents may be used singly or in combination; they may be prepared in simple aqueous solutions or suitable buffers.

Polyethylene glycol ter-octylphenyl ether is particularly useful for differential extraction by taking advantage of the low cloud point to separate membrane proteins from soluble proteins into two different phases. Extraction buffers may contain protease inhibitors, such as aprotinin, benzamidine, antipain, pepstatin, Phenylmethanesulfonyl fluoride (PMSF) and iodoacetamide.

Extracts are then assayed for nucleolin or PARP-1. In some cases, this may be achieved without removal of fragments of apoptotic bodies remaining after the extraction process. Preferably, nucleolin or PARP-1 is detected using an immunochemical assay technique. Various types of enzyme linked immunosorbent assays (ELISAs) to detect proteins, and these are applicable to nucleolin or PARP-1 detection. However, ELISA-like assays employing alternative labeling techniques may also be used. These include Radio Immunoassay (RIA), Fluorescent Immunoassay (FIA), Chemiluminescent Immunoassay (CMIA) and other non-enzyme linked antibody binding assays and procedures. Various assay formats including competitive (reagent limited) and immunometric assays may be used. In addition, heterogeneous assays and homogenous assays including agglutination assay, nephelometry and turbidimetry, enzyme-multiplied immunoassay technique (EMIT^{®}), and fluorescence polarization may be used, as well as other immunochemical assays.

The double antibody-sandwich ELISA technique is especially useful. The basic protocol for a double antibody-sandwich ELISA is as follows: A plate is coated with anti-nucleolin or PARP-1 antibodies (capture antibodies). The plate is then washed with a blocking agent, such as BSA, to block non-specific binding of proteins (antibodies or antigens) to the test plate. The test sample is then incubated on the plate coated with the capture antibodies. The plate is then washed, incubated with anti-nucleolin or PARP-1 antibodies, washed again, and incubated with a specific antibody-labeled conjugates and the signal appropriately detected.

In other ELISAs, proteins or peptides are immobilized onto a selected surface, the surface can have, or treated to have, an affinity for polypeptide attachment, such as the wells of a specially-treated polystyrene microtiter plates. After washing to remove incompletely adsorbed material, one would then generally desire to bind or coat with a nonspecific protein that is known to be antigenically neutral with anti-nucleolin or PARP-1 antibodies, such as BSA or casein, onto the well bottom. This step allows for blocking of nonspecific adsorption sites on the immobilizing surface and thus reduces the background caused by nonspecific binding of antibodies onto the surface. When the antibodies were created in an animal by conjugating a polypeptide to a protein (*e.g*., BSA), a different protein is usually used as a blocking agent, because of the possibility of the presence of antibodies to the blocking protein the antibody composition.

After binding of nucleolin or PARP-1 to the well, coating with a non-reactive material to reduce background, and washing to remove unbound material, the immobilizing surface is contacted with an anti-nucleolin or PARP-1 antibody composition in a manner conducive to immune complex (antigen/antibody) formation. Such conditions include diluting the antibody composition with diluents such as BSA, bovine γ globulin (BGG) and PBS/Polyoxyethylenesorbitan monolaurate. These added agents also assist in the reduction of nonspecific background signal. The layered antibody composition is then allowed to incubate for, *e.g*., from about two to four hours at 25° C to 37° C. Following incubation, the antibody composition-contacted surface is washed so as to remove non-immunocomplexed material. One washing procedure includes washing with a PBS/polyoxyethylenesorbitan monolaurate or borate buffer solution.

Following formation of specific immunocomplexes between the test sample and the antibody and subsequent washing, immunocomplex formation is detected using a second antibody having specificity for the anti-nucleolin or PARP-1 antibody. For detection, the secondary antibody is associated with detectable label, such as an enzyme or a fluorescent molecule.

### Western (immuno) blotting

Western blotting methods are well known (Ausubel, 1987). Generally, a protein sample is subjected to sodium dodecyl sulfate-polyacrylamide gel electrophoresis (SDS-PAGE) at such conditions as to yield an appropriate separation of proteins within the sample. The proteins are then transferred to a membrane (*e.g*., nitrocellulose, nylon, *etc*.) in such a way as to maintain the relative positions of the proteins to each other.

Visibly labeled proteins of known molecular weight may be included within a lane of the gel. These proteins serve as a control to insure adequate transfer of the proteins to the membrane, as well as molecular weight markers for determining the relative molecular weight of other proteins on the blot. Alternatively, unlabeled marker proteins are detected after transfer with Brilliant Blue (G or R; Sigma; St. Louis, MO) other protein dyes. After protein transfer, the membrane is submersed in a blocking solution to prevent nonspecific binding of the primary antibody.

The primary antibody, *e*.*g*., anti-nucleolin or PARP-1, may be labeled and the presence and molecular weight of the antigen may be determined by detecting the label at a specific location on the membrane. However, the primary antibody may not be labeled, and the blot is further reacted with a labeled second antibody. This secondary antibody is immunoreactive with the primary antibody; for example, the secondary antibody may be one to rabbit imunoglobulins and labeled with alkaline phosphatase.

### Detecting nucleolin:: Oligonucleotide-based methods

GROs and other oligonucleotides that recognize and bind nucleolin (Bates *et al*., 1999; Miller *et al*., 2000; Xu *et al*., 2001) can be used much the same way as antibodies are. Examples of suitable assays are given below. In some cases, incorporating the GRO nucleotides into larger nucleic acid sequences may be advantageous; for example, to facilitate binding of a GRO nucleic acid to a substrate without denaturing the nucleolin-binding site.

Useful GROs that bind nucleolin (and also have the biological property of inhibiting cancer cell growth) have been described (Bates *et al*., 1999; Miller *et al*., 2000; Xu *et al.,* 2001). They include those shown in Table 2. Control GROs are useful for detecting background signal levels.

**Table 2 Non-antisense GRO that bind nucleolin and non-binding controls^{1,2,3}**

| **GRO** | **Sequence** | **SEQ ID NO:** |
|---|---|---|
| GRO29A¹ | tttggtggtg gtggttgtgg tggtggtgg | 1 |
| GRO29-2 | tttggtggtg gtggttttgg tggtggtgg | 2 |
| GRO29-3 | tttggtggtg gtggtggtgg tggtggtgg | 3 |
| GRO29-5 | tttggtggtg gtggtttggg tggtggtgg | 4 |
| GRO29-13 | tggtggtggt ggt | 5 |
| GRO14C | ggtggttgtg gtgg | 6 |
| GRO15A | gttgtttggg gtggt | 7 |
| GRO15B² | ttgggggggg tgggt | 8 |
| GRO25A | ggttggggtg ggtggggtgg gtggg | 9 |
| GRO26B¹ | ggtggtggtg gttgtggtgg tggtgg | 10 |
| GRO28A | tttggtggtg gtggttgtgg tggtggtg | 11 |
| GRO28B | tttggtggtg gtggtgtggt ggtggtgg | 12 |
| GRO29-6 | ggtggtggtg gttgtggtgg tggtggttt | 13 |
| GRO32A | ggtggttgtg gtggttgtgg tggttgtggt gg | 14 |
| GRO32B | tttggtggtg gtggttgtgg tggtggtggt tt | 15 |
| GRO56A | ggtggtggtg gttgtggtgg tggtggttgt ggtggtggtg gttgtggtgg tggtgg | 16 |
| CRO² | tttcctcctc ctccttctcc tcctcctcc | 18 |
| GRO A | ttagggttag ggttagggtt aggg | 19 |
| GRO B | ggtggtggtg g | 20 |
| GRO C | ggtggttgtg gtgg | 21 |
| GRO D | ggttggtgtg gttgg | 22 |
| GRO E | gggttttggg | 23 |
| GRO F | ggttttggtt ttggttttgg | 24 |
| GRO G¹ | ggttggtgtg gttgg | 25 |
| GRO H¹ | ggggttttgg gg | 26 |
| GRO I¹ | gggttttggg | 27 |
| GRO J¹ | ggggttttgg ggttttgggg ttttgggg | 28 |
| GRO K¹ | ttggggttgg ggttggggtt gggg | 29 |
| GRO L¹ | gggtgggtgg gtgggt | 30 |
| GRO M¹ | ggttttggtt ttggttttgg ttttgg | 31 |
| GRO N² | tttcctcctc ctccttctcc tcctcctcc | 32 |
| GRO O² | cctcctcctc cttctcctcc tcctcc | 33 |
| GRO P² | tggggt | 34 |
| GRO Q² | gcatgct | 35 |
| GRO R² | gcggtttgcg g | 36 |
| GRO S² | tagg | 37 |
| GRO T | ggggttgggg tgtggggttg ggg | 38 |
| ¹Indicates a good plasma membrane nucleolin-binding GRO. ²Indicates a nucleolin control (non-plasma membrane nucleolin binding). ³GRO sequence without¹ or ² designations have some anti-proliferative activity. | | |

### Cytological-based approaches:

### Localization/labeling (relative of immuno-based localization/labeling assays)

The procedures outlined above for the immuno-based localization assays (such as immunofluorescence or FACS) are also applicable to those assays wherein the detecting reagent is a nucleolin-binding GRO. Modifications include those to prevent non-specific binding, using denatured DNA, such as from salmon sperm, instead of a protein such as BSA. For detection, similar labels as outlined above are also useful as long as the GRO can be derivatized or associated with the label in some form. For this purpose, biotin-avidin nucleic acid labeling systems are especially convenient, as are digoxigenin ones (Ausubel, 1987). The synthesis of biotinylated nucleotides has been described (Langer *et al*., 1981). Biotin, a water-soluble vitamin, can covalently attached to the C5 position of the pyrimidine ring via an alylamine linker arm; biotin non-covalently binds avidin or streptavidin, which can be easily labeled. Alternatively, biotin is added to oligonucleotides during synthesis by coupling to the 5'-hydroxyl of the terminal nucleotide. Digoxigenin-11-dUTP can be incorporated into DNA by either nick translation or random oligonucleotide-primed synthesis protocols. Digoxigenin is detected using labeled anti-digoxigenin antibodies. Convenient digoxigenin systems are commercially available (Roche Molecular Biochemicals; Indianapolis, IN). An example of a procedure using oligonucleotides to detect and localize proteins has been described by (Davis *et al*., 1998).

### Biochemical assay-based approaches:

GROs may also be used in a similar fashion as antibodies to detect nucleolin in biochemical approaches, as described above. For example, "Southwestern"-type blotting experiments may be performed with GROs (Bates *et al*., 1999; Miller *et al*., 2000). After apoptotic bodies have been appropriately extracted, the proteins are subjected to electrophoresis on polyacrylamide gels and transferred to a substrate, such as a polyvinlidene difluoride membrane. Proteins are denatured and renatured by washing for 30 minutes at 4° C with 6 M gaunidine-HCl, followed by washes in 3 M, 1.5 M and 0.75 M guanidine HCl in 25 mM HEPES (pH 7.9; 4 mM KCl/3 mM MgCl₂). After blocking non-specific binding sites with 5% non-fat dried milk in HEPES buffer, the labeled GRO is hybridized for 2 hours at 4° C in HEPES binding buffer supplemented with 0.25% NDM, 0.05% NP-40, 400 ng/ml salmon sperm DNA and 100 ng/ml of an unrelated mixed sequence oligonucleotide, such as tcgagaaaaa cectcctctc cttccttcct ctcca; SEQ ID NO:17. After washing with HEPES binding buffer, the signal is detected appropriately.

### Other methods:

### Arrays

### Arrays of immobilized nucleolin or PARP-1-binding reagents on chips

A chip is an array of regions containing immobilized molecules, separated by regions containing no molecules or immobilized molecules at a much lower density. For example, a protein chip may be prepared by applying nucleolin or PARP-1-binding antibodies; an "aptamer"-like chip may be prepared by applying nucleolin binding GROs. The remaining regions are left uncovered or are covered with inert molecules. The arrays can be rinsed to remove all but the specifically immobilized polypeptides or nucleic acids. In addition, chips may also be prepared containing multiple nucleolin-binding antibodies (Table 1A) or multiple anti-PARP-1 antibodies (Table 1B), nucleic acids (such as GROs; Table 2), or both, and may contain control antibodies and/or nucleic acids that are non-reactive with nucleolin and/or PARP-1. Such an array would allow for simultaneous test confirmation, duplication and internal controls.

Proteins, such as anti-nucleolin or PARP-1 antibodies, can be immobilized onto solid supports by simple chemical reactions, including the condensation of amines with carboxylic acids and the formation of disulfides. This covalent immobilization of proteins on inert substrates can prevent high background signals due to non-specific adsorption. Substrates derivatized with other molecules, such as biotin, are also useful when the protein to be immobilized is derivatized with avidin or streptavidin, or *vice-versa*. In some rare cases, especially when anti-nucleolin or PARP-1 antibody-encoding nucleic acid sequences are available, fusion polypeptides comprising anti-nucleolin or PARP-1 antibody may be advantageous for immobilization onto a substrate.

The surface may be any material to which the nucleolin or PARP-1 binding agent can be immobilized. For example, the surface may be metal, glass, ceramic, polymer, wood or biological tissue. The surface may include a substrate of a given material and a layer or layers of another material on a portion or the entire surface of the substrate. The surfaces may be any of the common surfaces used for affinity chromatography, such as those used for immobilization of glutathione for the purification of GST fusion polypeptides. The surfaces for affinity chromatography include, for example, sepharose, agarose, polyacrylamide, polystyrene and dextran. The surface need not be a solid, but may be a colloid, an exfoliated mineral clay, a lipid monolayer, a lipid bilayer, a gel, or a porous material.

The immobilization method desirably controls the position of the nucleolin or PARP-1 binding agent on the surface; for example, enabling the antigen binding portions of antibodies unattached to the substrate, while the non-antigen binding portions are rooted to the substrate. By controlling the position of individual reactant ligands, patterns or arrays of the ligands may be produced. The portions of the surface that are not occupied by the nucleolin or PARP-1-binding reagent do not allow non-specific adsorption of polypeptides or polynucleotides.

In this embodiment, a sample from a subject, for example, blood, is passed over a chip containing nucleolin or PARP-1-binding molecules. A biosensing device, such as machine that detects changes in surface plasmon resonance, is then used to detect bound nucleolin or PARP-1. BIAcore (Uppsala, Sweden) chips serve as examples of useful chips and detection machines.

### Prognostic assays

Diagnostic methods can furthermore be used to identify subjects having, or at risk of developing, a neoplasia at an early stage of disease development. Prognostic assays can be used to identify a subject having or at risk for developing a neoplasia, such as a subject who has a family history of harmful neoplasias, especially cancers. A method for identifying such an individual would include a test sample obtained from a subject, for example, a blood sample, and testing for the presence of apoptotic bodies containing nucleolin or PARP-1.

### Kits

Kits, containers, packs, or dispensers containing nucleolin or PARP-1 probes and detection reagents, together with instructions for administration, may be assembled. When supplied as a kit, the different components may be packaged in separate containers and admixed immediately before use. Such packaging of the components separately may permit long-term storage without losing the active components' functions.

Kits may also include reagents in separate containers that facilitate the execution of a specific test, such as diagnostic tests. For example, non-nucleolin-binding GROs may be supplied for internal negative controls, or nucleolin or PARP-1 and a nucleolin or PARP-1-binding reagent for internal positive controls. The components of a kit are an anti-nucleolin or PARP-1 agent used to probe for nucleolin, a control sample, and optionally a composition to detect nucleolin. Examples of anti-nucleolin or PARP-1 agents include an and-nucleolin or PARP-1 antibody (*e.g*., as shown in Tables 1A and 1B) or fragment thereof; if labeled, then a nucleolin or PARP-1-binding detection reagent is superfluous. A nucleolin-binding oligonucleotide (*e.g*., as shown in Table 2), which may be derivatized such that a second labeled reagent may bind (such as biotin). However, if a labeled GRO nucleic acid is provided, then a second labeled reagent is superfluous. Examples of detection reagents include: labeled secondary antibodies, for example, an anti-mouse polyclonal antibody made in donkey and then tagged with a fluorophore such as rhodamine, or a labeled reagent to detect oligonucleotides such as GROs; for example, avidin or streptavidin linked to horseradish peroxidase when the probe is biotinylated. Control components may include: normal serum from the animal in which a secondary antibody was made; a solution containing nucleolin or PARP-1 polypeptide or nucleolin binding oligonucleotide; a dot blot of nucleolin or PARP-1 protein to assay nucleolin or PARP-1-binding reagent reactivity; or fixed or preserved apoptotic bodies containing nucleolin. Other components may include buffers, fixatives, blocking solutions, microscope slides and/or cover slips or other suitable substrates for analysis, such as microtiter plates; detergent or detergent solutions or other permeabilizing reagents; miscellaneous reagents, protease inhibitors, various containers and miscellaneous tools and equipment to facilitate the assays.

In many cases, especially convenient kits may be assembled not only with the components listed above, but also with means for collecting a sample. For example, a needle and syringe may be provided to collect blood; additionally, sample containers containing buffers, preservatives, and/or anticoagulants may also be provided. Additionally, means to separate apoptotic bodies from whole cells may also be included. For example a syringe filter, a substrate (including beads) coated with a molecule to which cells bind but not apoptotic bodies, or test tubes suitable for centrifugation can be provided.

### (a) Containers or vessels

Reagents included in kits can be supplied in containers of any sort such that the life of the different components are preserved and are not adsorbed or altered by the materials of the container. For example, sealed glass ampules may contain lyophilized nucleolin or PARP-1 binding reagents (such as anti-nucleolin or PARP-1 antibodies or nucleolin or PARP-1-binding oligonucleotides) or buffers that have been packaged under a neutral, non-reacting gas, such as nitrogen. Ampules may consist of any suitable material, such as glass, organic polymers (*i*.*e*., polycarbonate, polystyrene, *etc*.), ceramic, metal or any other material typically employed to hold reagents. Other examples of suitable containers include simple bottles that may be fabricated from similar substances as ampules, and envelopes that may have foil-lined interiors, such as aluminum or alloy. Other containers include test tubes, vials, flasks, bottles, syringes, or the like. Containers may have a sterile access port, such as a bottle having a stopper that can be pierced by a hypodermic injection needle. Other containers may have two compartments that are separated by a readily removable membrane that upon removal permits the components to mix. Removable membranes may be glass, plastic, rubber, *etc.*

### (b) Instructional materials

Kits may also be supplied with instructional materials. Instructions may be printed on paper or other substrate and/or may be supplied as an electronic-readable medium, such as a floppy disc, CD-ROM, DVD-ROM, DVD, videotape, audio tape, *etc.* Detailed instructions may not be physically associated with the kit; instead, a user may be directed to an internet web site specified by the manufacturer or distributor of the kit, or supplied as electronic mail.

The following examples are intended to illustrate the present invention without limitation.

### EXAMPLES

### Example 1 Apoptosis of leukemia cells induced with UV radiation or a chemotherapy agent

Camptothecin (CPT; Sigma Co.; St. Louis, MO), an anti-neoplastic topoisomerase I (Top I) inhibitor, was dissolved in 0.5 % (v/v) dimethyl sulfoxide (DMSO)/PBS as stock solutions (stored at -20° C) and further diluted with water before use.

Human U937 cells (myeloid leukemia cell line, from American Type Culture Collection (ATCC); Manassas, VA) were grown in suspension in RPMI 1640 medium supplemented with 10% heat-inactivated (20 minutes at 65° C) fetal bovine serum (FBS), 100 U/ml penicillin, 100 µg/ml streptomycin at 37° C with 5% CO₂. For treatment with CPT, exponentially growing U937 cells were treated with 10 µM CPT for 24 hours. For UV irradiation, cells were plated at 5 x 10⁵ cells/ml in dishes (60 mm diameter). The cells were irradiated with UV-light by placing the plate (without a lid) directly in a Stratagene (La Jolla) UV Stratalinker and irradiating for 30 seconds at 254 nm. Some cells received 30 minutes pre-incubation with 1 mM 3-aminobenzamide (ABA); Sigma). Cells were then replaced in the incubator at 37° C for various times.

Apoptosis was observed using a DNA fragmentation assay (Facompre *et al*., 2001). In this assay, apoptosis is indicated by the appearance of a DNA "ladder", which is produced by endonuclease cleavage of chromosomal DNA into nucleosomal fragments. Apoptosis was detected as early as 1 hour following UV irradiation; a clear ladder was seen at 4 hours. Treatment with CPT also induced apoptosis, with the DNA ladder clearly observed at 24 hours.

### Example 2 Alternations of nucleolin and PARP-1 proteins in response to UV-induced apoptosis.

To examine apoptosis-induced changes in nucleolin and PARP-1, U937 cells, cultured as in Example 1, were irradiated with UV light, and cellular protein extracts were collected at different time points after irradiation.
Cells were harvested and washed twice with cold PBS. S-100 and nuclear extracts were prepared (Coqueret and Gascan, 2000). Briefly, 100 µl of ice-cold extraction buffer B (10 mM 4-(2-Hydroxyethyl)piperazine-1-ethanesulfonic acid (HEPES; pH7.9), 1.5 mM MgCl₂, 10 mM KCl, 1mM phenylmethylsulfonyl fluoride (PMSF), 1 µg/ml leupeptin, 1 µg/ml aprotinin) were added to the cells. After three cycles of freeze-thaw, S-100 extracts were recovered as supernatant following centrifugation at 12,000 rpm for 1 minute, and pellets containing nuclei were resuspended in 40 µl of buffer C (20 mM HEPES, pH7.9, 1.5mM MgCl₂, 420 mM KCl, 0.2 mM EDTA, 25% glycerol, 1 mM PMSF, 1 µg/ml leupeptin, 1 µg/ml aprotinin). Following 30 minutes incubation at 4° C, insoluble material was removed by centrifugation at 12,000 rpm for 5 minutes, and nuclear extracts were collected as supernatant. Extracts were either used immediately or frozen and stored at -80° C.
The concentration of extracted proteins was determined using the BioRad DC protein assay kit (BioRad; Hercules, CA). Samples (10µg) were incubated in sodium dodecyl sulfate (SDS)-loading buffer (100 mM Tris-HCl, pH 6.8, 200mM dithiothreitol (DTT), 4% SDS, 0.2% bromophenol blue, 20% glycerol) at 65° C for 15 minutes, and separated on 10% (for nucleolin detection) or 8% (for PARP-1) polyacrylamide-SDS gels, followed by electroblotting to polyvinylidene difluoride membranes (PVDF, BioRad). After blocking non-specific binding sites for 1 hour in 5% nonfat dried milk in PBST (0.1 % polyoxyethylene(20) sorbitan monolaurate (Tween® 20) in PBS), the membrane was incubated for 1 hour at room temperature or overnight at 4° C with primary antibody (1:1000 anti-nucleolin or anti-PARP-1; Anti-nucleolin antibody (mouse monoclonal IgG₁) and anti-PARP-1 antibody (mouse monoclonal IgG_{2A}) were from Santa Cruz Biotechnology; Santa Cruz; CA). After 3 washes in PBST, the membrane was incubated with horseradish peroxidase-conjugated goat anti-mouse antibody (Santa Cruz Biotechnology) for 45 minutes at room temperature and then washed 3 times in PBST. Bound antibodies were detected using enhanced chemiluminescence detection. Equal gel loading and transfer of proteins were confirmed by staining membranes with India ink (Bates *et al*., 1999).

Equal amounts of protein fractions were examined and consisted of nuclear extracts (soluble nuclear proteins) or S-100 extracts, which contain soluble proteins from the plasma membrane, cytosol and non-nuclear organelles. Immunoblot analysis showed that the U937 cells contained a high basal level of nucleolin for both S-100 and nuclear fractions and of PARP-1 (in nuclear extracts). PARP-1 was observed as predominantly the full-length product (118 kD), and nucleolin migrated on SDS-polyacrylamide gels at approximately 110 kD. An additional minor band was sometimes observed in the S-100 extracts blotted for nucleolin; the significance of this band is not known, but the mobility of the major S-100 nucleolin band corresponded to that of the nuclear fraction. Following irradiation with UV light, a profound decrease in the levels of S-100 nucleolin was observed, such that by 24 hours, this band was almost undetectable. Apoptosis also resulted in decreased levels of nuclear nucleolin. These nuclear changes were less pronounced than in the S-100 fraction, but occurred more rapidly and were already obvious by 2 hours after irradiation. By 72 hours after irradiation, levels of nuclear nucleolin had returned to baseline levels.

PARP-1 cleavage was an early event following UV-induced apoptosis. The active form of PARP-1 (118 KD protein) began to be cleaved to an inactive form (89 kD) by 2 hours after UV irradiation, and full-length PARP-1 was undetectable after 4 hours. Full-length PARP-1 did not begin to reappear until 48 hours after irradiation. Hence, PARP-1 cleavage was rapidly activated and preceded the disappearance of S-100 nucleolin by several hours. On the other hand, the inhibition of nuclear nucleolin levels appeared to occur roughly in parallel with cleavage of PARP-1.

### Example 3 Effect of the PARP-1 inhibitor, 3-aminobenzamide (3-ABA) on alternations of nucleolin and PARP-1 proteins in response to UV-induced apoptosis.

To investigate whether there was a direct relationship between cleavage of PARP-1 and UV-induced changes in nucleolin, experiments were performed in the absence or presence of a PARP-1 inhibitor, 3-aminobenzamide (3-ABA). Experimental conditions were as in Example 2. Some cells received 30 minutes pre-incubation with 1 mM 3-ABA before UV-irradiation. Immunoblot analysis of cellular protein extracts showed that 3-ABA-mediated abrogation of PARP-1 cleavage prevented the loss of nuclear nucleolin and drastically inhibited the disappearance of S-100 nucleolin.

Because PARP-1 is involved in both repair of DNA damage and induction of apoptosis, 3-ABA can both increase apoptotic cell death (by preventing repair) or decrease it (by preventing PARP-1 cleavage), depending on conditions. Therefore, the ability of 3-ABA to inhibit cell death under the conditions used here was examined. Cells were subjected to UV irradiation with or without 1 mM 3-ABA pretreatment. Untreated and irradiated cells were plated at 2 x 10⁴/ml in a 96-well plate. Viable cells were assessed using the MTT (3-[4,5-dimethylthiazol-2-yl]-2,5-diphenyl tetrazolium bromide) assay (Norgaard *et al.,* 2001) 48 hours after irradiation. Although the presence of 3-ABA could reduce UV-induced cell death, it did so only to a small degree, which did not seem to explain the strong inhibitory effects on nucleolin alterations.

To further investigate the potential relationship between nucleolin and PARP-1, co-immunoprecipitation experiments of U937 nuclear proteins were performed to determine if nucleolin and PARP-1 interact. Nuclear extracts were prepared as in Example 2 from untreated cells or at 8 hours after UV-light irradiation.

Immunoprecipitations were performed by incubating 200 µg extract with 2 µg PARP-1 antibody (mouse monoclonal IgG_{2A}, Santa Cruz Biotechnology) for 1 hour at 4° C, followed by adding protein A-agarose conjugate (20 µl; Sigma) and overnight incubation at 4° C on a rotator. Control immunoprecipitations were performed with normal mouse IgG (Santa Cruz Biotechnology) in place of primary antibody. The agarose beads were collected by centrifugation at 2500 rpm for 5 minutes and washed 4 times with RIPA buffer (PBS, 50 mM Tris-HCl pH 7.5, 0.5 M NaCl, 0.1 mM EDTA, 1% Nonidet^{®} P-40 (also known as Igepal CA 630; nonylphenyl-polyethylene glycol may also be used), 0.5% sodium deoxycholate, 0.1% SDS, 1 mM sodium fluoride, 10 mg/ml PMSF, 2 µM aprotinin, 100 mM sodium orthovanadate). The beads were resuspended in SDS-loading buffer, boiled for 3 minutes, and analyzed with SDS-PAGE. Immunoblot analysis was performed using nucleolin and PARP-1 antibodies as primary antibodies as described in Example 2. To analyze poly(ADP-ribosyl)ation of nucleolin, nucleolin antibody was used for immunoprecipitation, and anti-poly(ADP-Ribose) rabbit polyclonal antibody (1:2000; CALBIOCHEM; La Jolla, CA) was used to probe immunoblots.

Nucleolin was precipitated by the PARP-1 monoclonal antibody in both untreated and UV-treated cells, but was not precipitated in the absence of PARP-1 antibody or control IgG. Nucleolin was precipitated by both full-length and cleaved PARP-1.

PARP-1 is known to catalyze the,addition ofpoly(ADP-ribose) chains to substrate proteins in response to apoptotic stimuli, and 3-ABA can inhibit this enzymatic activity. Therefore, experiments were designed to investigate whether nucleolin was targeted for poly(ADP-ribosyl)ation in response to apoptosis. Previously, nuclear nucleolin had been reported to be a substrate for ADP-ribosylation in proliferating HeLa cells (Leitinger and Wesierska-Gadek, 1993). Nucleolin was immunoprecipitated from nuclear extracts derived from untreated or UV-irradiated U937 cells and immunoblotted using an antibody to poly(ADP)-ribose. In accord with the previous report, nucleolin was constitutively poly(ADP-ribosyl)ated in U937 cells. However, no significant changes in levels ofnucleolin-associated poly(ADP-ribose) between untreated and irradiated cells was observed.

### Example 4 Alternations of nucleolin and PARP-1 proteins in response to CPT-induced apoptosis.

To determine if the phenomena observed in Example 2 were specific to UV-irradiated cells or a general feature of apoptosis, protein changes in U937 cells treated with 10 µM CPT (prepared as in Example 1) for 24 hours were also examined. The U937 cells were cultured as in Example 2. Apoptosis-induced changes in nucleolin and PARP-1 were examined as in Example 2.

Apoptosis induced by CPT also caused a disappearance of nucleolin from the S-100 fraction and reduced the amount of nuclear nucleolin. However, these effects were less pronounced and occurred at later timepoints than for UV-irradiated cells. Similarly, the response of PARP-1 cleavage was also slightly delayed compared to the UV-treated cells, with only partial cleavage after 4 hours. In contrast to the irradiated cells, pre-incubation with 3-ABA produced only a small degree of protection from apoptosis-induced changes in nucleolin and PARP-1.

### Example 5 Redistributaion of nucleolin in cells undergoing apoptosis

The data clearly indicate reductions in the levels of nucleolin in both the nuclei and cytoplasm/plasma membrane of apoptotic cells. To investigate the fate of the nucleolin protein that disappears, the nuclei of apoptotic cells using immunofluorescent techniques to detect nucleolin was performed.

Cells were collected by centrifugation, washed twice with PBS, and placed on glass slides using a cytospinner. Samples were fixed in 4% paraformaldehyde in PBS for 15 minutes at room temperature and then permeabilized with 0.2% Triton X-100 in PBS for 10 minutes. After two washes with PBS, nonspecific antibody binding sites were blocked by 1 hour incubation at room temperature with 5% normal goat serum in PBS. After 3 washes with PBS, slides were incubated in primary antibody (1:100 anti-mAb IgG₁; Santa Cruz Biotechnology) for 1 hour at room temperature then washed 3 times in PBS. Samples were incubated with Alexa-488-labeled secondary antibodies (diluted 1:500 in blocking buffer) for 1 hour at room temperature. Slides were washed 3 times in PBS, were observed using an Olympus BX60F fluorescence microscope and photographed using an Olympus DP10 camera.

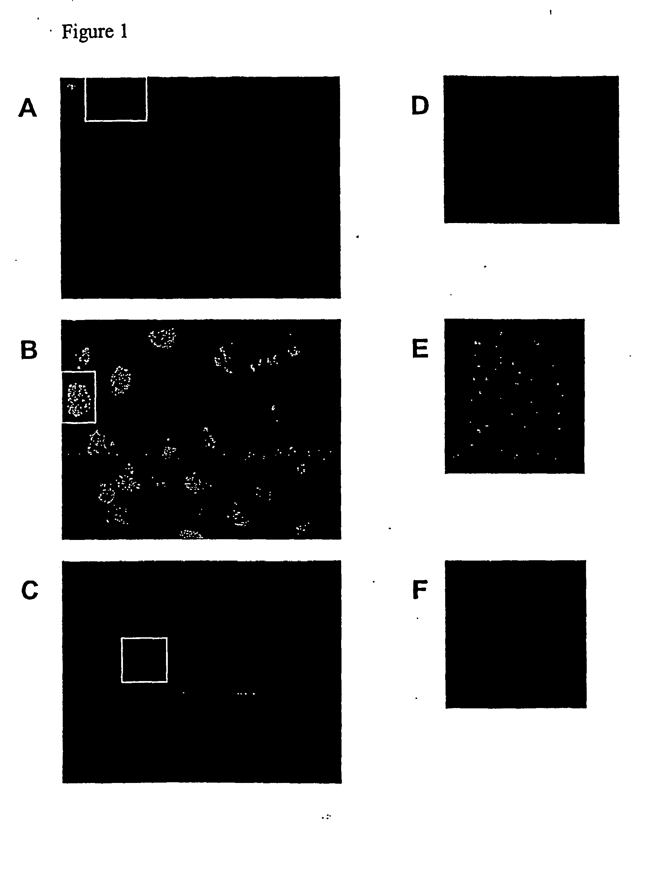

Untreated, UV-irradiated, and 10µM CPT-treated U937 cells were examined using the above staining method. Culture and treatment conditions were as in Example 2. Consistent with the data from Example 2, Figure 1 (panels A-C; areas marked by squares are shown in panels D-F to show single cells) shows that the overall intensity of nuclear nucleolin staining decreased slightly in response to UV irradiation or CPT treatment. Moreover, there was a dramatic redistribution of nucleolin in the apoptotic nuclei. In untreated cells, nucleolin was predominantly located in the intensely stained nucleoli (Figure 1, panels A and D), whereas 24 hours after UV irradiation, nucleolin was distributed throughout the nucleolplasm in a speckled pattern (Figure 1, panels B and E). In cells treated with CPT for 24 hours, nucleolar staining remained in some cells, but the majority of nuclei exhibited a distinct pattern of staining, similar to that seen in irradiated cells (Figure 1, panels C and F). It is notable that no cytoplasmic staining was seen, which is consistent with most of the S-100 nucleolin deriving from the plasma membrane.

### Example 6 Defection of nucleolin shed from U937 cells during apoptosis

The possibility that nucleolin was shed into the cell culture medium was examined by probing serum-free medium from cultures of U937 cells irradiated with UV light in the absence or presence of 3- ABA. Cell culture and treatment conditions were as in Example 2.

To prepare protein from the cell culture medium, the medium was replaced with serum-free medium, and cells were irradiated. Cells were then centrifuged at 1,200 rpm for 10 minutes. Culture medium was collected from untreated and treated cells at 2 hours and 4 hours following irradiation. The medium was filtered (syringe filters with PVDF membranes, Whatman; Clifton, NJ), and protein present in the medium was concentrated using Centricon (YM-30, Millipore, Bedford, MA) according the manufacturer's instructions.

Immunoblot analysis, also performed as in Example 2, showed that almost no nucleolin was detected in the medium of untreated cells, either with or without 3-ABA treatment. However, after UV irradiation, a clear band corresponding to full-length nucleolin was observed in the medium fraction, and the appearance of this band was inhibited by 3-ABA.

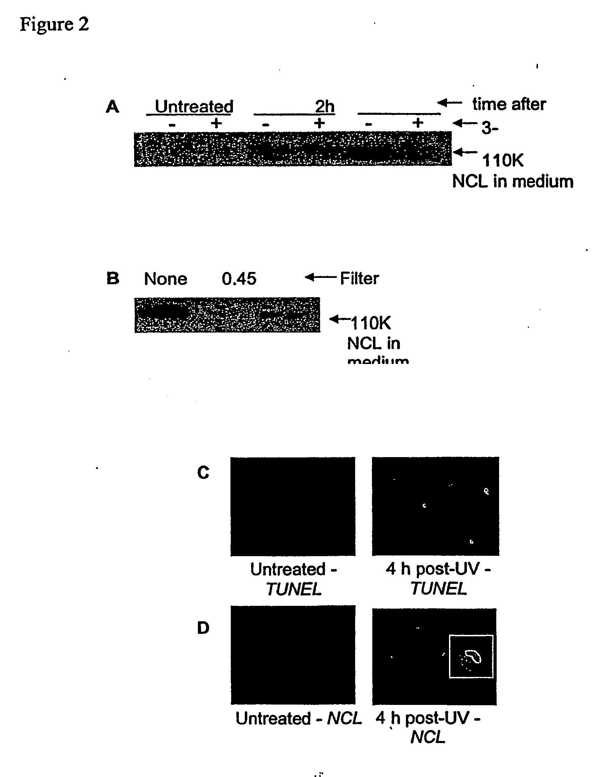

To determine if this nucleolin was derived from soluble protein or associated with cell-derived particles, the medium was pre-filtered before blotting. Filtration with a 0.2 µm filter caused the loss of nucleolin immunoreactivity, indicating that the nucleolin was not secreted as soluble protein; but rather, it was associated with particles of a size greater than 0.2 µm. Figure 2 shows that these results excluded the possibility that the immunoreactivity was entirely due to intact cells (which are >10 µm in diameter) that were not collected by centrifugation. Immunoblots show the presence of nucleolin in the medium of cells at different times after UV irradiation (Figure 2A), and the size of the nucleolin-containing particles was determined by prefiltering the medium using filters with pores of various sizes (Figure 2B). Immunoflurescence staining of the medium from untreated or UV-irradiated cells indicates that apoptosis induces the appearance of particles containing fragmented DNA typical of apoptotic bodies (Figure 2C, TUNEL staining) and nucleolin (Figure 2D, stained with anti-nucleolin). The inset to panel (Figure 2D) shows that some of these particles contain both nucleolin (anti-nucleolin stainin, periphery of center) and DNA (propidium iodide staining, center of body); staining overlaps as indicated by the circled regions. These particles were of a size that is consistent with the "apoptotic bodies" that are often observed in apoptotic cultured cells and in vivo in tissues undergoing apoptosis (Gautier *et al*., 1999; Kerr *et al.,* 1972; Schmidt-Acevedo *et al.,* 2000).

### Example 7 Detection of nucleolin and DNA in apoptotic bodies by nucleolin immunoreactivity and TUNEL staining

Samples from the medium of untreated and UV-irradiated U937 cells were prepared and analyzed them by terminal deoxynuclotidyltransferase (Tdt) - mediated dUTP nick-end labeling (TUNEL) staining for fragmented DNA (Gavrieli *et al*., 1992) and immunofluorescence staining of nucleolin. Cell culture and treatment conditions were as described in Exampe 6. At 2 hours and 4 hours after treatment, medium was collected and placed onto glass slides. The presence of nucleolin in the apoptotic bodies was detected by immunofluorescence staining using the same procedure described for cells in Example 5.

Slides containing the culture medium of apoptotic cells were prepared as above. After washing with PBS and incubating in permeabilization solution (0.1% Polyethylene glycol mono [4-(1,1,3,3-tetramethylbutyl)phenyl] ether (Triton X-100^{®}), 0.1% sodium citrate) for 2 minutes on ice, the slides were washed twice with PBS and dried. 50 µl of TUNEL reaction mixture (Roche; Basel, Switzerland) was added to each sample. The slides were then incubated in the dark in a humidified chamber for 60 minutes at 37° C and washed 3 times with PBS. The slides were then observed as in Example 5.

Figure 2 (panels C and D) shows the results of these studies. The apoptosis-induced bodies specifically appeared following UV irradiation and were strongly stained for both nucleolin and DNA fragmentation. To determine if nucleolin and DNA co-existed in the same particles, double staining for nucleolin and DNA (propidium iodide) was performed. Some, but not all, of the apoptotic bodies that stained positive for nucleolin also stained positive for the presence of DNA; an example is shown in the inset to Figure 2D. The nucleolin-positive bodies appeared as early as 1 hour following irradiation and were clearly seen at 4 hours. This timing paralleled the observation of nucleolin in the medium, the appearance of the DNA ladder, and the loss of nuclear nucleolin (Example 6), but preceded the loss of the plasma membrane nucleolin (Example 2).

### REFERENCES

Ausubel, F.M. 1987. Current protocols in molecular biology. Greene Publishing Associates ;
J. Wiley, order fulfillment, Brooklyn, N: Y.
Media, Pa. 2 v. (loose-leaf) pp.
Babkes, M., A. Ranford, and K. Yaeger. 2001. Sputum trap manifold with nested caps. US Patent No. 6,325,785.
Bandman, O., H. Yue, N. Corley, and P. Shah. 1999. Human nucleolin-like protein. US Patent No. 5,932,475.
Bates, P.J., J.B. Kahlon, S.D. Thomas, J.O. Trent, and D.M. Miller. 1999. Antiproliferative activity of G-rich oligonucleotides correlates with protein binding. J Biol Chem. 274:26369-77.
Biscotti, C.V., and W.R. Hart. 1998. Apoptotic bodies: a consistent morphologic feature of endocervical adenocarcinoma in situ. Am J Surg Pathol. 22:434-9.
Byrne, C.J. 1986. Laboratory tests : implications for nursing care. Addison-Wesley Pub. Co., Health Sciences Division, Menlo Park, Calif. xxi, 756 p. pp.
Callebaut, C., J. Blanco, N. Benkirane, B. Krust, E. Jacotot, G. Guichard, N. Seddiki, J. Svab, E. Dam, S. Muller, J.P. Briand, and A.G. Hovanessian. 1998. Identification of V3 loop-binding proteins as potential receptors implicated in the binding of HIV particles to CD4(+) cells. J Biol Chem. 273:21988-97.
Choi, N.G., J.H. Sohn, H.W. Park, and T.Y. Jung. 1999. Apoptosis and nuclear shapes in benign prostate hyperplasia and prostate adenocarcinoma: comparison with and relation to Gleason score. Int J Urol. 6:13-8.
Coqueret, O., and H. Gascan. 2000. Functional interaction of STAT3 transcription factor with the cell cycle inhibitor p21WAF1/CIP1/SDI1. J Biol Chem. 275:18794-800.
Davis, K.A., Y. Lin, B. Abrams, and S.D. Jayasena. 1998. Staining of cell surface human CD4 with 2'-F-pyrimidine-containing RNA aptamers for flow cytometry. Nucleic Acids Res. 26:3915-24.
Derenzini, M. 2000. The AgNORs. Micron. 31:117-20.
Facompre, M., J.F. Goossens, and C. Bailly. 2001. Apoptotic response of HL-60 human leukemia cells to the antitumor drug NB-506, a glycosylated indolocarbazole inhibitor of topoisomerase 1. Biochem Pharmacol. 61:299-310.
Gautier, F., D. Lassort, M.T. Le Cabellec, F. Videau, L. Minet, K. Meflah, G. Pradal, and J. Harb. 1999. Production and characterisation of a monoclonal antibody specific for apoptotic bodies derived from several tumour cell lines. J Immunol Methods. 228:49-58.
Gavrieli, Y., Y. Sherman, and S.A. Ben-Sasson. 1992. Identification of programmed cell death in situ via specific labeling of nuclear DNA fragmentation. J Cell Biol. 119:493-501*.*
Ginisty, H., H. Sicard, B. Roger, and P. Bouvet. 1999. Structure and functions of nucleolin. J Cell Sci. 112 (Pt 6):761-72.
Goding, J.W. 1996. Monoclonal antibodies : principles and practice. Academic Press, London; San Diego. xv, 492 pp.
Harlow, E., and D. Lane. 1988. Antibodies : a laboratory manual. Cold Spring Harbor Laboratory, Cold Spring Harbor, NY. xiii, 726 pp.
Harlow, E., and D. Lane. 1999. Using antibodies : a laboratory manual. Cold Spring Harbor Laboratory Press, Cold Spring Harbor, N.Y. xiv, 495 pp.
Holdenrieder, S., P. Stieber, H. Bodenmuller, M. Busch, G. Fertig, H. Furst, A. Schalhorn, N. Schmeller, M. Untch, and D. Seidel. 2001a. Nucleosomes in serum of patients with benign and malignant diseases. Int J Cancer. 95:114-20.
Holdenrieder, S., P. Stieber, H. Bodenmuller, M. Busch, J. Von Pawel, A. Schalhorn, D. Nagel, and D. Seidel. 2001b. Circulating nucleosomes in serum. Ann N Y Acad Sci. 945:93-102.
Holdenrieder, S., P. Stieber, H. Bodenmuller, G. Fertig, H. Furst, N. Schmeller, M. Untch, and D. Seidel. 2001 c. Nucleosomes in serum as a marker for cell death. Clin Chem Lab Med. 39:596-605.
Kerr, J.F., A.H. Wyllie, and A.R. Currie. 1972. Apoptosis: a basic biological phenomenon with wide-ranging implications in tissue kinetics. Br J Cancer. 26:239-57.
Kim, C.S., B.B. Berkley, W.M. Abraham, and A. Wanner. 1982. A micro double capillary method for rheologic measurements of lower airway secretions. Bull Eur Physiopathol Respir. 18:915-27.
King, M., and D. Speert. 2002. Use of dextran and other polysaccharides to improve mucous clearance. US Patent 6,339,075.
Langer, P.R., A.A. Waldrop, and D.C. Ward. 1981. Enzymatic synthesis of biotin-labeled polynucleotides: novel nucleic acid affinity probes. Proc Natl Acad Sci U S A. 78:6633-7.
Leitinger, N., and J. Wesierska-Gadek. 1993. ADP-ribosylation of nucleolar proteins in HeLa tumor cells. J Cell Biochem. 52:153-8.
Lichtenstein, A.V., H.S. Melkonyan, L.D. Tomei, and S.R. Umansky. 2001. Circulating nucleic acids and apoptosis. Ann N Y Acad Sci. 945:239-49.
Martin, S.J., and D.R. Green. 1995. Protease activation during apoptosis: death by a thousand cuts? Cell. 82:349-52.
McNicol, A.M., and J.A. Richmond. 1998. Optimizing immunohistochemistry: antigen retrieval and signal amplification. Histopathology. 32:97-103.
Miller, D.M., P. Bates, and J. Trent. 2000. Antiproliferative activity of G-rich oligonucleotides and method of using same to bind nucleolin. WO 00/61597.
Norgaard, J.M., L.H. Olesen, G. Olesen, K. Meyer, J.S. Kristensen, K. Bendix, B. Pedersen, E. Kjeldsen, and P. Hokland. 2001. FAB M4 and high CD14 surface expression is associated with high cellular resistance to Ara-C and daunorubicin: implications for clinical outcome in acute myeloid leukaemia. Eur J Haematol. 67:221-9.
Orfao, A., and A. Ruiz-Arguelles. 1996. General concepts about cell sorting techniques. Clin Biochem. 29:5-9.
Pinton, P., D. Ferrari, E. Rapizzi, F.D. Di Virgilio, T. Pozzan, and R. Rizzuto. 2001. The Ca2+ concentration of the endoplasmic reticulum is a key determinant of ceramide-induced apoptosis: significance for the molecular mechanism of Bcl-2 action. Embo J. 20:2690-701.
Robinson, J.M., and D.D. Vandre. 2001. Antigen retrieval in cells and tissues: enhancement with sodium dodecyl sulfate. Histochem Cell Biol. 116:119-30.
Rubin, B., and M. Newhouse. 1999. Use of surface active agents to promote mucus clearance. US Patent 5,925,334.
Schimmer, A.D., D.W. Hedley, L.Z. Penn, and M.D. Minden. 2001. Receptor- and mitochondrial-mediated apoptosis in acute leukemia: a translational view. Blood. 98:3541-53.
Schmidt-Acevedo, S., B. Perez-Romano, and A. Ruiz-Arguelles. 2000. 'LE cells' result from phagocytosis of apoptotic bodies induced by antinuclear antibodies. J Autoimmun. 15:15-20.
Scovassi, A.I., and G.G. Poirier. 1999. Poly(ADP-ribosylation) and apoptosis. Mol Cell Biochem. 199:125-37.
Siman, R., D. Bozyczko-Coyne, S. Meyer, and R. Bhat. 2000. Method for detecting cell apoptosis. US Patent No. 6,048,703.
Sohn, J.H., D.H. Kim, N.G. Choi, Y.E. Park, and J.Y. Ro. 2000. Caspase-3/CPP32 immunoreactivity and its correlation with frequency of apoptotic bodies in human prostatic carcinomas and benign nodular hyperplasias. Histopathology. 37:555-60.
Sorokina, E.A., and J.G. Kleinman. 1999. Cloning and preliminary characterization of a calcium-binding protein closely related to nucleolin on the apical surface of inner medullary collecting duct cells. J Biol Chem. 274:27491-6.
Srivastava, M., and H.B. Pollard. 1999. Molecular dissection of nucleolin's role in growth and cell proliferation: new insights. Faseb J. 13:1911-22.
Sutton, V.R., J.E. Davis, M. Cancilla, R.W. Johnstone, A.A. Ruefli, K. Sedelies, K.A. Browne, and J.A. Trapani. 2000. Initiation of apoptosis by granzyme B requires direct cleavage of bid, but not direct granzyme B-mediated caspase activation. J Exp Med. 192:1403-14.
Thornberry, N.A., and Y. Lazebnik. 1998. Caspases: enemies within. Science. 281:1312-6.
Tormanen, U., A.K. Eerola, P. Rainio, K. Vahakangas, Y. Soini, R. Sormunen, R. Bloigu, V.P. Lehto, and P. Paakko. 1995. Enhanced apoptosis predicts shortened survival in non-small cell lung carcinoma. Cancer Res. 55:5595-602.
Tuteja, R., and N. Tuteja. 1998. Nucleolin: a multifunctional major nucleolar phosphoprotein. Crit Rev Biochem Mol Biol. 33:407-36.
Wyllie, A.H., J.F. Kerr, and A.R. Currie. 1980. Cell death: the significance of apoptosis. Int Rev Cytol. 68:251-306.
Xu, X., F. Hamhouyia, S.D. Thomas, T.J. Burke, A.C. Girvan, W.G. McGregor, J.O. Trent, D.M. Miller, and P.J. Bates. 2001. Inhibition of DNA replication and induction of S phase cell cycle arrest by G-rich oligonucleotides. J Biol Chem. 276:43221-30.
Zou, H., W. Henzel, and X. Wang. 2001. Apaf-1 an activator of caspase-3. US Patent No. 6,291,643.

## Claims

1. A method of detecting apoptosis, comprising:
detecting at least one of nucleolin and poly(ADP-ribose) polymerase in or from apoptotic bodies in a sample, wherein said sample is a sample from which cells have been removed.

2. The method of Claim 1, wherein the sample is prepared by removing cells from blood, tissue, tissue culture medium or sputum.

3. The method of Claim 1, wherein the sample is serum or plasma.

4. The method of Claim 1, wherein the detecting comprises membrane disruption.

5. The method of Claim 1, wherein the detecting is detecting nucleolin, and the detecting nucleolin comprises detecting a nucleolin binding molecule-nucleolin complex.

6. The method of Claim 5, wherein the nucleolin binding molecule comprises an anti-nucleolin antibody.

7. The method of Claim 6, wherein the antibody is selected from the group consisting of p7-1A4, sc-8031, sc-9893, sc-9892, 4E2 and 3G4B2 antibodies.

8. The method of Claim 5, wherein the nucleolin binding molecule comprises a guanosine-rich oligonucleotide.

9. The method of Claim 8, wherein the guanosine-rich oligonucleotide comprises an oligonucleotide having a nucleotide sequence of SEQ ID NO: 1-7, 9-17, 19-30 or 31.

10. The method of Claim 9, wherein the guanosine-rich oligonucleotide comprises an oligonucleotide having a nucleotide sequence of SEQ ID NO: 1, 10, 25-30 or 31.

11. The method of Claim 1, wherein the detecting is detecting poly(ADP-ribose) polymerase; and the detecting poly(ADP-ribose) polymerase comprises detecting a poly(ADP-ribose) polymerase binding molecule- poly(ADP-ribose) polymerase complex.

12. The method of Claim 11, wherein the poly(ADP-ribose) polymerase binding molecule comprises an anti-poly(ADP-ribose) polymerase antibody.

13. The method of Claim 12, wherein the antibody is selected from the group consisting of sc-1562, sc-8007, sc-1561, sc-1561-Y and sc-7150 antibodies.

14. A method according to any preceding claim, wherein the method is for detecting excessive apoptosis in a subject.

15. The method of Claim 14, wherein the subj ect is suspected of having a disease selected from the group consisting of Acquired Immunodeficiency Syndrome, a neurodegenerative disease, an ischemic injury, an autoimmune disease, a tumour, a cancer, a viral infection, an acute inflammatory condition and sepsis.

16. The method of Claim 14, wherein the subject is suspected of having cancer.

17. The method of Claim 16, wherein the cancer is selected from the group consisting of endocervical adenocarcinoma, prostatic carcinoma, breast cancer, leukemia and non-small cell lung carcinoma.

18. A method of determining if a compound induces apoptosis, comprising:
contacting a cell with the compound; and
detecting apoptosis by the method according to any of Claims 1-13.

19. A method according to Claim 1, wherein the method is for detecting apoptosis in a cell culture.

20. The method of Claim 19, wherein the cell culture is grown in a bioreactor.

## Patentansprüche

1. Verfahren zum Nachweisen von Apoptose, das umfasst:
Nachweisen wenigstens eines von Nucleolin und Poly(ADP-Ribose) Polymerase in oder aus apoptotischen Körpern in einer Probe, wobei die Probe eine Probe ist, aus der Zellen entfernt worden sind.

2. Verfahren nach Anspruch 1, wobei die Probe durch Entfernen von Zellen aus Blut, Gewebe, Gewebekulturmedium oder Sputum hergestellt wird.

3. Verfahren nach Anspruch 1, wobei die Probe Serum oder Plasma ist.

4. Verfahren nach Anspruch 1, wobei das Nachweisen eine Membranzersetzung umfasst.

5. Verfahren nach Anspruch 1, wobei das Nachweisen das Nachweisen von Nucleolin ist, und wobei das Nachweisen von Nucleolin das Nachweisen eines nucleolinbindenden Molekül-Nucleolin-Komplexes umfasst.

6. Verfahren nach Anspruch 5, wobei das nucleolinbindende Molekül einen Antinucleolin-Antikörper umfasst.

7. Verfahren nach Anspruch 6, wobei der Antikörper ausgewählt wird aus der Gruppe bestehend aus p7-1A4-, sc-8031-, sc-9893-, sc-9892-, 4E2- und 3G4B2-Antikörpern.

8. Verfahren nach Anspruch 5, wobei das nucleolinbindende Molekül ein guanosinreiches Oligonucleotid aufweist.

9. Verfahren nach Anspruch 8, wobei das guanosinreiche Oligonucleotid ein Oligonucleotid mit einer Nucleotidsequenz der SEQ ID Nr. 1-7, 9-17, 19-30 oder 31 umfasst.

10. Verfahren nach Anspruch 9, wobei das guanosinreiche Oligonucleotid ein Oligonucleotid mit einer Nucleotidsequenz der SEQ ID Nr. 1, 10, 25-30 oder 31 umfasst.

11. Verfahren nach Anspruch 1, wobei das Nachweisen das Nachweisen von Poly(ADP-Ribose)-Polymerase ist, und wobei das Nachweisen von Poly(ADP-Ribose)-Polymerase das Nachweisen eines Poly(ADP-Ribose)- Polymerasebindenden Molekül-Poly(ADP-Ribose)-Polymerase-Komplexes umfasst.

12. Verfahren nach Anspruch 11, wobei das Poly(ADP-Ribose)-Polymerase-bindende Molekül einen Anti-Poly(ADP-Ribose) Polymerase-Antikörper umfasst.

13. Verfahren nach Anspruch 12, wobei der Antikörper aus der Gruppe bestehend aus sc-1562-, sc-8007-, sc-1561-, sc-1561-Y- und sc-7150-Antikörpern ausgewählt wird.

14. Verfahren nach einem der vorhergehenden Ansprüche, wobei das Verfahren zum Nachweisen übermäßiger Apoptose in einer Testperson vorgesehen ist.

15. Verfahren nach Anspruch 14, wobei von der Testperson angenommen wird, dass sie eine Krankheit ausgewählt aus der Gruppe bestehend aus erworbenem Immunschwächesyndrom, neurodegenerativer Krankheit, ischämischer Verletzung, Autoimmunerkrankung, Tumor, Krebs, Virusinfektion, akutem Entzündungszustand und Sepsis hat.

16. Verfahren nach Anspruch 14, wobei von der Testperson angenommen wird, dass sie Krebs hat.

17. Verfahren nach Anspruch 16, wobei der Krebs ausgewählt wird aus der Gruppe bestehend aus endozervikalem Adenokarzinom, Prostatakarzinom, Brustkrebs, Leukämie und nichtkleinzelligem Lungenkarzinom.

18. Verfahren zum Bestimmen, ob eine Verbindung Apoptose induziert, das umfasst:
Kontaktieren einer Zelle mit der Verbindung; und
Nachweisen von Apoptose durch das Verfahren nach einem der Ansprüche 1 bis 13.

19. Verfahren nach Anspruch 1, wobei das Verfahren zum Ermitteln von Apoptose in einer Zellkultur vorgesehen ist.

20. Verfahren nach Anspruch 19, wobei die Zellkultur in einem Bioreaktor gezüchtet wird.

## Revendications

1. Procédé de détection de l'apoptose, comprenant :
la détection d'au moins un élément parmi la nucléoline et la poly(ADP-ribose) polymérase dans ou à partir de corps apoptotiques dans un échantillon, ledit échantillon étant un échantillon à partir duquel des cellules ont été retirées.

2. Procédé selon la revendication 1, dans lequel l'échantillon est préparé en retirant des cellules du sang, de tissus, d'un milieu de culture tissulaire ou de la salive.

3. Procédé selon la revendication 1, dans lequel l'échantillon est du sérum ou du plasma.

4. Procédé selon la revendication 1, dans lequel la détection comprend la rupture des membranes.

5. Procédé selon la revendication 1, dans lequel la détection est la détection de la nucléoline, et la détection de la nucléoline comprend la détection d'un complexe molécule de liaison à la nucléoline - nucléoline.

6. Procédé selon la revendication 5, dans lequel la molécule de liaison à la nucléoline comprend un anticorps anti-nucléoline.

7. Procédé selon la revendication 6, dans lequel l'anticorps est choisi dans le groupe constitué des anticorps p7-1A4, sc-8031, sc-9893, sc-9892, 4E2 et 3G4B2.

8. Procédé selon la revendication 5, dans lequel la molécule de liaison à la nucléoline comprend un oligonucléotide riche en guanosine.

9. Procédé selon la revendication 8, dans lequel l'oligonucléotide riche en guanosine comprend un oligonucléotide ayant une séquence de nucléotides de SEQ ID NO : 1 - 7, 9 - 17, 19 - 30 ou 31.

10. Procédé selon la revendication 9, dans lequel l'oligonucléotide riche en guanosine comprend un oligonucléotide ayant une séquence de nucléotides de SEQ ID NO : 1, 10, 25 - 30 ou 31.

11. Procédé selon la revendication 1, dans lequel la détection est la détection de la poly(ADP-ribose) polymérase ; et la détection de la poly(ADP-ribose) polymérase comprend la détection d'un complexe molécule de liaison à la poly(ADP-ribose) polymérase - poly(ADP-ribose) polymérase.

12. Procédé selon la revendication 11, dans lequel la molécule de liaison à la poly(ADP-ribose) polymérase comprend un anticorps anti-poly(ADP-ribose) polymérase.

13. Procédé selon la revendication 12, dans lequel l'anticorps est choisi dans le groupe constitué des anticorps sc-1562, sc-8007, sc-1561, sc-1561-Y et sc-7150.

14. Procédé selon l'une quelconque des revendications précédentes, le procédé étant destiné à la détection d'une apoptose excessive chez un sujet.

15. Procédé selon la revendication 14, dans lequel le sujet est suspecté d'avoir une maladie choisie dans le groupe constitué du syndrome d'immunodéficience acquise, d'une maladie neurodégénérative, d'une blessure ischémique, d'une maladie autoimmune, d'une tumeur, d'un cancer, d'une infection virale, d'un état inflammatoire aigu et d'une sepsie.

16. Procédé selon la revendication 14, dans lequel le sujet est suspecté d'avoir un cancer.

17. Procédé selon la revendication 16, dans lequel le cancer est choisi dans le groupe constitué de l'adénocarcinome endocervical, du carcinome de la prostate, du cancer du sein, de la leucémie et du carcinome bronchique non à petites cellules.

18. Procédé consistant à déterminer si un composé induit l'apoptose, comprenant
la mise en contact une cellule avec le composé ; et
le détection de l'apoptose par le procédé selon l'une quelconque des revendications 1 à 13.

19. Procédé selon la revendication 1, le procédé étant destiné à la détection de l'apoptose dans une culture cellulaire.

20. Procédé selon la revendication 19, dans lequel la culture cellulaire est effectuée dans un bioréacteur.


### SEQUENCE LISTING

<110> Bates, Paula J
   Mi, Yingchang
<120> A NEW METHOD FOR THE DIAGNOSIS AND PROGNOSIS OF MALIGNANT DISEASES
<130> 09799910-0035
<150> 60/392,143
<151> 2002-06-26
<160> 38
<170> Patent In version 3.2
<210> 1
<211> 29
<212> DNA
<213> Artificial sequence
<220>
<223> synthetic oligonucleotide
<400> 1
   tttggtggtg gtggttgtgg tggtggtgg 29
<210> 2
<211> 29
<212> DNA
<213> Artificial sequence
<220>
<223> synthetic oligonucleotide
<400> 2
   tttggtggtg gtggttttgg tggtggtgg 29
<210> 3
<211> 29
<212> DNA
<213> Artificial sequence
<220>
<223> synthetic oligonucleotide
<400> 3
   tttggtggtg gtggtggtgg tggtggtgg 29
<210> 4
<211> 29
<212> DNA
<213> Artificial sequence
<220>
<223> synthetic oligonucleotide
<400> 4
   tttggtggtg gtggtttggg tggtggtgg 29
<210> 5
<211> 13
<212> DNA
<213> Artificial sequence
<220>
<223> synthetic oligonucleotide
<400> 5
   tggtggtggt ggt 13
<210> 6
<211> 14
<212> DNA
<213> Artificial sequence
<220>
<223> synthetic oligonucleotide
<400> 6
   ggtggttgtg gtgg 14
<210> 7
<211> 15
<212> DNA
<213> Artificial sequence
<220>
<223> synthetic oligonucleotide
<400> 7
   gttgtttggg gtggt 15
<210> 8
<211> 15
<212> DNA
<213> Artificial sequence
<220>
<223> synthetic oligonucleotide
<400> 8
   ttgggggggg tgggt 15
<210> 9
<211> 25
<212> DNA
<213> Artificial sequence
<220>
<223> synthetic oligonucleotide
<400> 9
   ggttggggtg ggtggggtgg gtggg 25
<210> 10
<211> 26
<212> DNA
<213> Artificial sequence
<220>
<223> synthetic oligonucleotide
<400> 10
   ggtggtggtg gttgtggtgg tggtgg 26
<210> 11
<211> 28
<212> DNA
<213> Artificial sequence
<220>
<223> synthetic oligonucleotide
<400> 11
   tttggtggtg gtggttgtgg tggtggtg 28
<210> 12
<211> 28
<212> DNA
<213> Artificial sequence
<220>
<223> synthetic oligonucleotide
<400> 12
   tttggtggtg gtggtgtggt ggtggtgg 28
<210> 13
<211> 29
<212> DNA
<213> Artificial sequence
<220>
<223> synthetic oligonucleotide
<400> 13
   ggtggtggtg gttgtggtgg tggtggttt 29
<210> 14
<211> 32
<212> DNA
<213> Artificial sequence
<220>
<223> synthetic oligonucleotide
<400> 14
   ggtggttgtg gtggttgtgg tggttgtggt gg 32
<210> 15
<211> 32
<212> DNA
<213> Artificial sequence
<220>
<223> synthetic oligonucleotide
<400> 15
   tttggtggtg gtggttgtgg tggtggtggt tt 32
<210> 16
<211> 56
<212> DNA
<213> Artificial sequence
<220>
<223> synthetic oligonucleotide
<400> 16
   ggtggtggtg gttgtggtgg tggtggttgt ggtggtggtg gttgtggtgg tggtgg 56
<210> 17
<211> 35
<212> DNA
<213> Artificial sequence
<220>
<223> synthetic oligonucleotide
<400> 17
   tcgagaaaaa ctctcctctc cttccttcct ctcca 35
<210> 18
<211> 29
<212> DNA
<213> Artificial Sequence
<220>
<223> synthetic oligonucleotide
<400> 18
   tttcctcctc ctccttctcc tcctcctcc 29
<210> 19
<211> 24
<212> DNA
<213> Artificial Sequence
<220>
<223> synthetic oligonucleotide
<400> 19
   ttagggttag ggttagggtt aggg 24
<210> 20
<211> 11
<212> DNA
<213> Artificial Sequence
<220>
<223> synthetic oligonucleotide
<400> 20
   ggtggtggtg g 11
<210> 21
<211> 14
<212> DNA
<213> Artificial Sequence
<220>
<223> synthetic oligonucleotide
<400> 21
   ggtggttgtg gtgg 14
<210> 22
<211> 15
<212> DNA
<213> Artificial Sequence
<220>
<223> synthetic oligonucleotide
<400> 22
   ggttggtgtg gttgg 15
<210> 23
<211> 10
<212> DNA
<213> Artificial Sequence
<220>
<223> synthetic oligonucleotide
<400> 23
   gggttttggg 10
<210> 24
<211> 20
<212> DNA
<213> Artificial Sequence
<220>
<223> synthetic oligonucleotide
<400> 24
   ggttttggtt ttggttttgg 20
<210> 25
<211> 15
<212> DNA
<213> Artificial sequence
<220>
<223> synthetic oligonucleotide
<400> 25
   ggttggtgtg gttgg 15
<210> 26
<211> 12
<212> DNA
<213> Artificial sequence
<220>
<223> synthetic oligonucleotide
<400> 26
   ggggttttgg gg 12
<210> 27
<211> 10
<212> DNA
<213> Artificial sequence
<220>
<223> synthetic oligonucleotide
<400> 27
   gggttttggg 10
<210> 28
<211> 28
<212> DNA
<213> Artificial sequence
<220>
<223> synthetic oligonucleotide
<400> 28
   ggggttttgg ggttttgggg ttttgggg 28
<210> 29
<211> 24
<212> DNA
<213> artificial sequence
<220>
<223> synthetic oligonucleotide
<400> 29
   ttggggttgg ggttggggtt gggg 24
<210> 30
<211> 16
<212> DNA
<213> artificial sequence
<220>
<223> synthetic oligonucleotide
<400> 30
   gggtgggtgg gtgggt 16
<210> 31
<211> 26
<212> DNA
<213> artificial sequence
<220>
<223> synthetic oligonucleotide
<400> 31
   ggttttggtt ttggttttgg ttttgg 26
<210> 32
<211> 29
<212> DNA
<213> artificial sequence
<220>
<223> synthetic oligonucleotide
<400> 32
   tttcctcctc ctccttctcc tcctcctcc 29
<210> 33
<211> 26
<212> DNA
<213> artificial sequence
<220>
<223> synthetic oligonucleotide
<400> 33
   cctcctcctc cttctcctcc tcctcc 26
<210> 34
<211> 6
<212> DNA
<213> artificial sequence
<220>
<223> synthetic oligonucleotide
<400> 34
   tggggt 6
<210> 35
<211> 7
<212> DNA
<213> artificial sequence
<220>
<223> synthetic oligonucleotide
<400> 35
   gcatgct 7
<210> 36
<211> 11
<212> DNA
<213> Artificial sequence
<220>
<223> synthetic oligonucleotide
<400> 36
   gcggtttgcg g 11
<210> 37
<211> 4
<212> DNA
<213> artificial sequence
<220>
<223> synthetic oligonucleotide
<400> 37
   tagg 4
<210> 38
<211> 23
<212> DNA
<213> artificial sequence
<220>
<223> synthetic oligonucleotide
<400> 38
   ggggttgggg tgtggggttg ggg 23